# EUROPEAN PATENT APPLICATION

(11) **EP 1 541 173 A1**
(43) Date of publication of application: **15.06.2005**
(21) Application number: 03791417.3
(22) Date of filing: 29.08.2003
(51) Int. Cl.: A61K 45/00, A61P 3/10, C07K 7/06

(54) **METHOD OF DEGRADING TRANSCRIPTIONAL FACTORS OF SACCHAROMETABOLISM-ASSOCIATED GENE, METHOD OF INHIBITING THE DEGRADATION AND DEGRADATION INHIBITOR**

(30) Priority: 30.08.2002 JP 2002254973; 31.03.2003 JP 2003096370; 31.03.2003 JP 2003096371; 31.03.2003 JP 2003096372
(71) Applicant: DAIICHI PHARMACEUTICAL CO., LTD., Chuo-ku, Tokyo 103-8234 (JP)
(72) Inventor: DOI, H., c/o Celestar Lexico-Sciences, Inc., Mihama-ku, Chiba-shi, Chiba 261-8501 (JP); KUDO, G., c/o Daiichi Phar Co. Ltd.Tokyo R&D Cntr., Edogawa-ku, Tokyo 134-863 (JP)
(74) Representative: De Clercq, Ann
(86) International application number: PCT/JP2003/011046
(87) International publication number: WO 2004/019983

(57) **Abstract**

Based on the finding that m-calpain or µ-calpain degrades hepatocyte nuclear factor 4α (HNF-4α), hepatocyte nuclear factor 1α (HNF-1α) and insulin promoter factor 1 (IPF-1), which form transcription factor networks involved in expression of glucose metabolism-related genes in pancreatic β cells, the following have been provided: a method for degradation of these transcription factors; a method for inhibiting the degradation and an agent for inhibiting the degradation; a method for enhancing production of the gene product of a gene on which these factors act as transcription factors and an agent for enhancing the same; an agent for preventing and/or treating a disease attributable to the degradation of these transcription factors and a method for preventing and/or treating the disease; a method for identifying a compound that inhibits the degradation of these transcription factors by calpain; a compound obtained by the identification method; and a reagent kit including calpain, these transcription factors, polynucleotides encoding these factors, and a vector containing the polynucleotides.

## Description

### TECHNICAL FIELD

The present invention relates to a method for degradation of transcription factors of glucose metabolism-related genes, an agent for degradation of the same, a method for inhibiting the degradation of the same, and an agent for inhibiting the degradation of the same. More particularly, the present invention relates to a method for degradation of hepatocyte nuclear factor 4α (hereunder, referred to as "HNF-4α"), hepatocyte nuclear factor 1α (hereunder, referred to as "HNF-1α") and insulin promoter factor 1 (hereunder, referred to as "IPF-1"), an agent for degradation of the same, a method for inhibiting the degradation of the same, and an agent for inhibiting the degradation of the same. Specifically, the present invention relates to a method for degradation of HNF-4α, HNF-1α and IPF-1, and an agent for degradation of the same, wherein the method and agent comprise using calpain, preferably m-calpain or µ-calpain. The present invention further relates to a method for inhibiting the degradation of HNF-4α, HNF-1α and IPF-1 caused by calpain, preferably m-calpain or µ-calpain, and an agent for inhibiting the degradation of the same. The present invention also relates to a method for enhancing production of a gene product of a gene on which at least one of HNF-4α, HNF-1α and IPF-1 acts as a transcription factor, and an agent for enhancing production of the same. The present invention further relates to a method for preventing and/or treating a disease attributable to the degradation of at least one of HNF-4α, HNF-1α and IPF-1, such as, for example, diabetes, and an agent for preventing and/or treating the same. The present invention still further relates to a method for identifying a compound that inhibits the degradation of HNF-4α, HNF-1α and IPF-1 caused by calpain, and a compound identified by the identification method. The present invention further relates to a reagent kit, wherein the reagent kit comprises calpain, HNF-4α, HNF-1α, IPF-1, a polynucleotide encoding any of these, or a vector containing the polynucleotide.

### BACKGROUND ART

Calpain (EC 3.4.22.17), a calcium-dependent cysteine protease, is an enzyme that restrictively cleaves a protein to alter the structure and function thereof. A large number of isozymes of calpain are known, which are classified according to their structural characteristics, tissue localization, calcium requirement and the like, and comprise a super family.

m-Calpain, also referred to as "calpain 2," is one member of the calpain super family and is expressed in many tissues (Non-patent Reference 1). m-Calpain is activated by about 1 mM concentration of calcium to express enzyme activity.

Similarly to m-calpain, µ-calpain, also referred to as "calpain 1," is expressed in many tissues (Non-patent Reference 1 and 2). µ-Calpain, which has a low calcium requirement in comparison to m-calpain, is activated by a calcium concentration of around several tens of µM to express enzyme activity.

Many transcription factors such as p53 and retinoid X receptor (RXR) have been reported as proteins that are degraded by m-calpain and µ-calpain (Non-patent Reference 3 to 5). Proteins that are degraded by calpain have amino acid motifs that are preferentially cleaved by calpain (Non-patent Reference 6). For example, cleavage occurs between a tyrosine residue (Tyr), methionine residue (Met) or arginine residue (Arg) that follows a hydrophobic amino acid residue such as a leucine residue (Leu) or valine residue (Val), and the following amino acid residue. A number of calpain inhibitors are currently commercially available. The peptide N-Acetyl-Leu-Leu-Met-CHO has an amino acid sequence that contains this kind of cleavage motif and is known as a competitive inhibitor of m-calpain (Calbiochem, Inc., Non-patent Reference 7). Further, N-Acetyl-Leu-Leu-Nle-CHO is known as a competitive inhibitor of µ-calpain (Calbiochem, Inc., Non-patent Reference 8). In addition, the irreversible calpain inhibitors Z-Leu-Leu-Tyr-CH₂F (Non-patent Reference 9), Mu-Val-HPh-CH₂F (Non-patent Reference 10) and Leu-Leu-Pro-chloromethyl ketone (Non-patent Reference 11), the reversible calpain inhibitor 4-fluorophenylsulfonyl-Val-Leu-CHO (Non-patent Reference 12), and the like, are commercially available (Calbiochem, Inc.).

Since calpain is involved in the regulation of cellular function, dysregulation of calpain activity, or a defect in calpain gene or the like, causes various diseases. For example, tissue calpain activity increases in several animal models of diabetes (Non-patent Reference 13 and 14). Because the insulin secretory response to glucose in the pancreatic islet was enhanced by a calpain inhibitor, it has been suggested that calpain is involved in the regulation of the secretion and action of insulin (Non-patent Reference 15). A relationship between mutation of the calpain 10 gene and the incidence of type 2 diabetes mellitus has also been indicated (Non-patent Reference 16 and 17).

It has also been suggested that m-calpain and µ-calpain are involved in traumatic brain injury, Alzheimer's disease, cerebral hemorrhage and cataracts (Non-patent Reference 2).

Meanwhile, it is known that HNF-4α, HNF-1α and IPF-1 each have a function as a transcription factor and bind to promoters or enhancers of various genes to activate transcription of the gene. It is believed that, for example, they form a transcription factor network in pancreatic β cells (Non-patent Reference 18) to regulate expression of glucose metabolism-related genes, such as the glucose transporter 2 (hereunder, sometimes abbreviated as "GLUT2") gene, insulin gene and glucokinase gene.

HNF-4α, which is a nuclear receptor and is expressed in pancreatic β cells, liver, kidney and small intestine, is known to be a transcription factor of various genes that encode substances that are involved in the metabolism of cholesterol, fatty acids, glucose and the like, or in the development and differentiation of the liver. In the aforementioned transcription factor network in pancreatic β cells, HNF-4α functions as a positive regulator of HNF-1α in particular, and regulates expression of glucose metabolism-related genes such as the insulin gene, GLUT2 gene and glucokinase gene (Non-patent Reference 18 to 23). It had been thought that the action of HNF-4α with respect to insulin gene expression was an indirect action through an increase in the level of HNF-1α. However, it has been reported that HNF-4α binds to a cis-element that was newly discovered within the insulin gene promoter and directly regulates the expression of insulin (Non-patent Reference 24).

It has been clarified that the HNF-4α gene is a responsive geneof hereditary type 2 diabetes mellitus, MODY1 (maturity-onset diabetes of the young 1) (Non-patent Reference 25 and 26).

Further, a few human genes having a natural mutation at a HNF-4α binding site have been identified. A gene encoding HNF-1α mentioned above is one of the genes for which mutants having a mutation at a HNF-4α binding site within the promoter were found. Because of the mutation at a HNF-4α binding site in the HNF-1α promoter, HNF-4α does not bind to the promoter, which results in a decrease in the expression of HNF-1α and the induction of hereditary type 2 diabetes mellitus, MODY3 (Non-patent Reference 27).

Other examples of such genes include genes respectively encoding factor VII and factor IX, which are factors involved in blood coagulation. A mutation at an HNF-4α binding site in a promoter of the factor IX gene is a cause of haemophilia (Non-patent Reference 28 and 29). Further, a mutation at an HNF-4α binding site in a promoter of the factor VII gene is observed in patients suffering from severe factor VII deficiency (Non-patent Reference 30).

HNF-1α, which is also referred to as transcription factor 1 (TCF-1) and is expressed in pancreatic β cells, liver, kidney, and the like, is known to regulate gene expression of many liver specific genes such as the albumin gene, fibrinogen gene and α1-antitrypsin gene. HNF-1α regulates transcription of IPF-1 and HNF-4α in the aforementioned transcription factor network in pancreatic β cells (Non-patent Reference 21). Data has also been disclosed which suggests that HNF-1α regulates expression of glucose metabolism-related genes such as the GLUT2 gene, insulin gene and the like (Non-patent Reference 22). For example, HNF-1α bound to a promoter region of the GLUT2 gene and enhanced the transcription activity thereof (Non-patent Reference 22). HNF-1α also bound to an A3 region of a promoter of the insulin gene and enhanced the transcription activity thereof (Non-patent Reference 31). It is also reported that dominant negative HNF-1α decreased insulin secretion in a pancreatic β cell line (Non-patent Reference 32).

It has been clarified that the HNF-1α gene is a responsive geneof hereditary type 2 diabetes mellitus, MODY3 (maturity-onset diabetes of the young 3).

A relationship between HNF-1α and liver adenoma has also been indicated. For example, it was shown that inactivation of HNF-1α due to an amino mutation is a cause of liver adenoma (Non-patent Reference 34). Further, a marked liver enlargement due to increased proliferation of hepatocyte neoplasia has been observed in HNF-1α knockout mice (Non-patent Reference 35). Liver adenoma is a tumor with a risk of transforming into hepatocellular carcinoma. It is therefore believed that inactivation of HNF-1α induces liver adenoma and subsequently hepatocellular carcinoma.

IPF-1 is also referred to as "pancreas/duodenum homeobox 1 (PDX-1)", and is expressed in β cells and δ cells of pancreas. It has been reported that an IPF-1 binding site is present in a promoter region, P2, of HNF-4α, and a mutation thereof correlates with the onset of diabetes (Non-patent Reference 36). Further, data has been disclosed which suggests that IPF-1 regulates expression of glucose metabolism-related genes such as the GLUT2 gene (Non-patent Reference 37), insulin gene (Non-patent Reference 38) and glucokinase gene (Non-patent Reference 39). IPF-1 acts directly on the GLUT2 gene and is involved in the expression thereof (Non-patent Reference 37).

It has also been clarified that the IPF-1 gene is a responsive geneof hereditary type 2 diabetes mellitus, MODY 4 (maturity-onset diabetes of the young 4) (Non-patent Reference 40).

The References cited in the specification are listed as follows:
Patent Reference 1: International Publication No. WO 01/67299.
Non-patent Reference 1: Sorimachi, H., "SEIKAGAKU", 2000, Vol. 72, No. 11, p. 1297-1315.
Non-patent Reference 2: Huang, Y., et al., "Trends in Molecular Medicine", 2001, Vol. 7, p. 355-362.
Non-patent Reference 3: Matsusima-Nishiwaki, R. et al., "Biochemical and Biophysical Research Communications", 1996, Vol. 225, p. 946-951.
Non-patent Reference 4: Pariat, M., et al., "Molecular and Cellular Biology", 1997, Vol. 17, p. 2806-2815.
Non-patent Reference 5: Watt, F., et al., "Nucleic Acids Research", 1993, Vol. 21, p. 5092-5100.
Non-patent Reference 6: Sasaki, T., et al., "Journal of Biological Chemistry", 1984, Vol. 259, p. 12489-12494.
Non-patent Reference 7: Ravid, T., et al., "Journal of Biological Chemistry", 2000, Vol. 275, p. 35840-35847.
Non-patent Reference 8: Debiasi, R. L., et al., "Journal of Virology", 1999, Vol. 73, p. 695-701.
Non-patent Reference 9: Dutt, P., et al., "FEBS Letter", 1998, Vol. 436, p. 367-371.
Non-patent Reference 10: Esser, R. E., et al., "Arthritis and Rheumatism", 1994, Vol. 37, p. 236-247.
Non-patent Reference 11: Sasaki, T. et al., "Journal of Biochemistry", 1986, Vol. 99, p. 173-179.
Non-patent Reference 12: Nath R., et al., "Biochemical and Biophysical Research Communications", 2000, Vol. 274, p. 16-21.
Non-patent Reference 13: Brooks, B. A., et al., "American Journal of Physiology", 1983, Vol. 244, No. 3, p. C175-181.
Non-patent Reference 14: Kobayashi, S., et al., "Endocrinologia Japonica", 1989, Vol. 36, No. 6, p. 833-844.
Non-patent Reference 15: Sreeman, S. K., et al., "Diabetes", 2001, Vol. 50, p. 2013-2020.
Non-patent Reference 16: Horikawa, Y., et al., "Nature Genetics", 2000, Vol. 26, p. 163-175.
Non-patent Reference 17: Baier, L. J., et al., "Journal of Clinical Investigation", 2000, Vol. 106, p. R69-73.
Non-patent Reference 18: Shih, D. Q., et al., "Proceedings of the National Academy of Sciences of the United States ofAmerica", 2001, Vol. 98, p. 14189-14191.
Non-patent Reference 19: "BIO Clinica", 2002, Vol. 17, p. 410-414.
Non-patent Reference 20: Stoffel, M., et al., "Proceedings of the National Academy of Sciences of the United States of America", 1997, Vol. 94, p. 13209-13214.
Non-patent Reference 21: Shih, D. Q., et al., "Diabetes", 2001, Vol. 50, p. 2472-2480.
Non-patent Reference 22: Ban, N., et al., "Diabetes", 2002, Vol. 51, p. 1409-1418.
Non-patent Reference 23: Cah, J. Y., et al., "Experimental and Molecular Medicine", 2001, Vol. 33, No. 2, p. 59-63.
Non-patent Reference 24: Bartoov-Shifman, R., et al., "Journal of Biological Chemistry", 2002, Vol. 277, No. 29, p. 25914-25919.
Non-patent Reference 25: Ryffel, G. U., et al., "Journal of Molecular Endrocrinology", 2001, Vol. 27, p. 11-29.
Non-patent Reference 26: "RINSHOU BYOURI [Jpn. J. Clin. Pathol.]", 2001, Vol. 49, No. 2, p. 161-164.
Non-patent Reference 27: Gragnoli, C., et al., "Diabetes", 1997, Vol. 46, p. 1648-1651.
Non-patent Reference 28: Sladek, F. M., et al., "In Nuclear Receptors and Genetic Disease", 2001, p. 309-361, Eds. TB Burris & ERB McCabe, San Diego; Academic Press.
Non-patent Reference 29: Marlene, J. R., et al., "Proceedings of the National Academy of Sciences of the United States of America", 1992, Vol. 89, p. 6300-6303.
Non-patent Reference 30: Carew, J. A., et al., "BLOOD", 2000, Vol. 15, p. 4370-4372.
Non-patent Reference 31: Okita, K., et al., "Biochemical Biophysical Research Communications", 1999, Vol. 263, p. 566-569.
Non-patent Reference 32: Wang, H., et al., "EMBO Journal", 1998, Vol. 17, No. 22, p. 6701-6713.
Non-patent Reference 33: Yamagata, K., et al., "Nature", 1996, Vol. 384, p. 455-457.
Non-patent Reference 34: Bluteau, O., et al., "Nature genetics", 2002, Vol. 32, p. 312-315.
Non-patent Reference 35: Pontoglio, M., et al., "Cell", 1996, Vol. 84, p. 575-585.
Non-patent Reference 36: Thomas, H., et al., "Human Molecular Genetics", 2001, Vol. 10, No. 19, p. 2089-2097.
Non-patent Reference 37: Waeber, G., et al., "Molecular Endocrinology", 1996, Vol. 10, p. 1327-1334.
Non-patent Reference 38: Ohlsson, H., et al., "EMBO Journal", 1993, Vol. 12, p. 4251-4259.
Non-patent Reference 39: Watada, H., et al., "Diabetes", 1996, Vol. 45, p. 1478-1488.
Non-patent Reference 40: Stoffers, D. A., et al., "Nature genetics", 1997, Vol. 17, p. 138-141.
Non-patent Reference 41: Ulmer, K. M. "Science", 1983, Vol. 219, p. 666-671.
Non-patent Reference 42: "PEPUTIDO GOUSEI" (Japan), Maruzen Co., Ltd., 1975.
Non-patent Reference 43: "Peptide Synthesis" (USA), Interscience, 1996.
Non-patent Reference 44: Bjorklund, A., et al., "Diabetes", 2000, Vol. 49, p. 1840-1848.
Non-patent Reference 45: Maruyama, K., et al., "International Journal of Molecular Medicine", 2000, Vol. 5, p. 269-273.

### DISCLOSURE OF THE INVENTION

The present inventors carried out various studies to discover proteins that interact with calpain, for the purpose of providing means for preventing and/or treating diseases attributable to the degradation of the proteins by calpain. As a result, we predicted in-silico that calpain interacts with HNF-4α, a transcription factor, and further experimentally demonstrated the degradation of HNF-4α by calpain. We also discovered that HNF-1α and IPF-1, which form a transcription factor network in pancreatic β cells with HNF-4α, are degraded by calpain, to thus complete the present invention.

That is, one aspect of the present invention relates to a method for degrading a transcription factor of a glucose metabolism-related gene, wherein the method comprises making calpain coexist with the transcription factor of the glucose metabolism-related gene in the presence of calcium.

Another aspect of the present invention relates to a method for degrading a transcription factor of a glucose metabolism-related gene, wherein the method comprises changing the degree of degradation of the transcription factor of the glucose metabolism-related gene by calcium concentration.

A further aspect of the present invention relates to a method for degrading a transcription factor of a glucose metabolism-related gene, wherein the method comprises making m-calpain and/or µ-calpain coexist with the transcription factor of the glucose metabolism-related gene in the presence of calcium.

A further aspect of the present invention relates to any of the foregoing degradation methods, wherein the transcription factor of the glucose metabolism-related gene is at least one member selected from the group consisting of hepatocyte nuclear factor 4α, hepatocyte nuclear factor 1α and insulin promoter factor 1.

A still further aspect of the present invention relates to a method for degrading hepatocyte nuclear factor 4α (HNF-4α), wherein the method comprises making m-calpain and/or µ-calpain coexist with HNF-4α in the presence of calcium.

A further aspect of the present invention relates to a method for degrading hepatocyte nuclear factor 1α (HNF-1α), wherein the method comprises making m-calpain and/or µ-calpain coexist with HNF-1α in the presence of calcium.

A further aspect of the present invention relates to a method for degrading insulin promoter factor 1 (IPF-1), wherein the method comprises making m-calpain and/or µ-calpain coexist with IPF-1 in the presence of calcium.

A still further aspect of the present invention relates to a method for inhibiting the degradation of a transcription factor of a glucose metabolism-related gene, wherein the method comprises inhibiting calpain activity.

A further aspect of the present invention relates to a method for inhibiting the degradation of a transcription factor of a glucose metabolism-related gene, wherein the method comprises inhibiting the cleavage of the transcription factor of the glucose metabolism-related gene by calpain.

A further aspect of the present invention relates to a method for inhibiting the degradation of a transcription factor of a glucose metabolism-related gene, wherein the method comprises inhibiting the binding of calpain to the transcription factor of the glucose metabolism-related gene.

A further aspect of the present invention relates to a method for inhibiting the degradation of a transcription factor of a glucose metabolism-related gene, wherein the method comprises treating an *in vitro* sample containing at least calpain and the transcription factor of the glucose metabolism-related gene with a substance that inhibits calpain activity.

A further aspect of the present invention relates to a method for inhibiting the degradation of a transcription factor of a glucose metabolism-related gene, wherein the method comprises treating a cell expressing at least calpain and the transcription factor of the glucose metabolism-related gene with a substance that inhibits calpain activity.

A further aspect of the present invention relates to the preceding method for inhibiting the degradation of a transcription factor of a glucose metabolism-related gene, wherein the cell is a cell that is carried by a mammal.

A further aspect of the present invention relates to the preceding method for inhibiting the degradation of a transcription factor of a glucose metabolism-related gene, wherein the cell that is carried by a mammal is a pancreatic β cell.

A still further aspect of the present invention relates to any of the foregoing methods for inhibiting the degradation of a transcription factor of a glucose metabolism-related gene, wherein the substance that inhibits calpain activity is one or more substances selected from the group consisting of an antibody that recognizes calpain, an antibody that recognizes the transcription factor of the glucose metabolism-related gene, and a calpain inhibitor.

A further aspect of the present invention relates to the preceding method for inhibiting the degradation of a transcription factor of a glucose metabolism-related gene, wherein the calpain inhibitor is N-Acetyl-Leu-Leu-Met-CHO, N-Acetyl-Leu-Leu-Nle-CHO, Z-Leu-Leu-Tyr-CH₂F, Mu-Val-HPh-CH₂F, 4-fluorophenylsulfonyl-Val-Leu-CHO, Leu-Leu-Phe-CH₂Cl or Z-Val-Phe-CHO.

A further aspect of the present invention relates to any of the foregoing methods for inhibiting the degradation of a transcription factor of a glucose metabolism-related gene, wherein the substance that inhibits calpain activity is a peptide containing at least one amino acid sequence of a calpain-recognized cleavage site in the transcription factor of the glucose metabolism-related gene.

A further aspect of the present invention relates to any of the foregoing methods for inhibiting the degradation of a transcription factor of a glucose metabolism-related gene, wherein the substance that inhibits calpain activity is a peptide that comprises three or more consecutive amino acid residues from the amino acid sequence set forth in any of SEQ ID NOS: 1 to 3 in the sequence listing and contains at least one amino acid sequence of a calpain-recognized cleavage site in the transcription factor of the glucose metabolism-related gene.

A still further aspect of the present invention relates to the foregoing method for inhibiting the degradation of a transcription factor of a glucose metabolism-related gene, wherein the calpain-recognized cleavage site in the transcription factor of the glucose metabolism-related gene is selected from the group consisting of Leu-Tyr, Leu-Met, Leu-Arg, Val-Tyr, Val-Met and Val-Arg.

A further aspect of the present invention relates to any of the foregoing methods for inhibiting the degradation of a transcription factor of a glucose metabolism-related gene, wherein calpain is m-calpain and/or µ-calpain.

A further aspect of the present invention relates to any of the foregoing methods for inhibiting the degradation of a transcription factor of a glucose metabolism-related gene, wherein the transcription factor of the glucose metabolism-related gene is at least one member selected from the group consisting of hepatocyte nuclear factor 4α, hepatocyte nuclear factor 1α and insulin promoter factor 1.

Another aspect of the present invention relates to a method for inhibiting the degradation of hepatocyte nuclear factor 4α, wherein the method comprises inhibiting the activity of m-calpain and/or µ-calpain.

A further aspect of the present invention relates to a method for inhibiting the degradation of hepatocyte nuclear factor 1α, wherein the method comprises inhibiting the activity of m-calpain and/or µ-calpain.

A further aspect of the present invention relates to a method for inhibiting the degradation of insulin promoter factor 1, wherein the method comprises inhibiting the activity of m-calpain and/or µ-calpain.

Another aspect of the present invention relates to an agent for degrading a transcription factor of a glucose metabolism-related gene, wherein the agent contains an effective dose of calpain as an active ingredient.

A further aspect of the present invention relates to the preceding agent for degrading a transcription factor of a glucose metabolism-related gene, wherein calpain is m-calpain and/or µ-calpain.

A further aspect of the present invention relates to the foregoing agent for degrading a transcription factor of a glucose metabolism-related gene, wherein the transcription factor of the glucose metabolism-related gene is at least one member selected from the group consisting of hepatocyte nuclear factor 4α, hepatocyte nuclear factor 1α and insulin promoter factor 1.

A still further aspect of the present invention relates to an agent for degrading hepatocyte nuclear factor 4α, wherein the agent contains an effective dose of m-calpain and/or µ-calpain as an active ingredient.

A further aspect of the present invention relates to an agent for degrading hepatocyte nuclear factor 1α, wherein the agent contains an effective dose of m-calpain and/or µ-calpain as an active ingredient.

A further aspect of the present invention relates to an agent for degrading insulin promoter factor 1, wherein the agent contains an effective dose of m-calpain and/or µ-calpain as an active ingredient.

Another aspect of the present invention relates to an agent for inhibiting the degradation of a transcription factor of a glucose metabolism-related gene, wherein the agent inhibits calpain activity.

A further aspect of the present invention relates to an agent for inhibiting the degradation of a transcription factor of a glucose metabolism-related gene, wherein the agent inhibits the cleavage of the transcription factor of the glucose metabolism-related gene by calpain.

A still further aspect of the present invention relates to an agent for inhibiting the degradation of a transcription factor of a glucose metabolism-related gene, wherein the agent inhibits the binding of calpain to the transcription factor of the glucose metabolism-related gene.

A further aspect of the present invention relates to an agent for inhibiting the degradation of a transcription factor of a glucose metabolism-related gene, wherein the agent contains an effective dose of a substance that inhibits calpain activity as an active ingredient.

A further aspect of the present invention relates to the preceding agent for inhibiting the degradation of a transcription factor of a glucose metabolism-related gene, wherein the substance that inhibits calpain activity is one or more substances selected from the group consisting of an antibody that recognizes calpain, an antibody that recognizes the transcription factor of the glucose metabolism-related gene, and a calpain inhibitor.

A further aspect of the present invention relates to the preceding agent for inhibiting the degradation of a transcription factor of a glucose metabolism-related gene, wherein the calpain inhibitor is N-Acetyl-Leu-Leu-Met-CHO, N-Acetyl-Leu-Leu-Nle-CHO, Z-Leu-Leu-Tyr-CH₂F, Mu-Val-HPh-CH₂F, 4-fluorophenylsulfonyl-Val-Leu-CHO, Leu-Leu-Phe-CH₂Cl or Z-Val-Phe-CHO.

A further aspect of the present invention relates to the foregoing agent for inhibiting the degradation of a transcription factor of a glucose metabolism-related gene, wherein the substance that inhibits calpain activity is a peptide containing at least one amino acid sequence of a calpain-recognized cleavage site in the transcription factor of the glucose metabolism-related gene.

A further aspect of the present invention relates to the foregoing agent for inhibiting the degradation of a transcription factor of a glucose metabolism-related gene, wherein the substance that inhibits calpain activity is a peptide that comprises three or more consecutive amino acid residues from the amino acid sequence set forth in any of SEQ ID NOS: 1 to 3 in the sequence listing and contains at least one amino acid sequence of a calpain-recognized cleavage site in the transcription factor of the glucose metabolism-related gene.

A further aspect of the present invention relates to the preceding agent for inhibiting the degradation of a transcription factor of a glucose metabolism-related gene, wherein the calpain-recognized cleavage site in the transcription factor of the glucose metabolism-related gene is selected from the group consisting of Leu-Tyr, Leu-Met, Leu-Arg, Val-Tyr, Val-Met and Val-Arg.

A still further aspect of the present invention relates to any of the foregoing agents for inhibiting the degradation of a transcription factor of a glucose metabolism-related gene, wherein calpain is m-calpain and/or µ-calpain.

A further aspect of the present invention relates to any of the foregoing degradation inhibitory agents, wherein the transcription factor of the glucose metabolism-related gene is at least one member selected from the group consisting of hepatocyte nuclear factor 4α, hepatocyte nuclear factor 1α, and insulin promoter factor 1.

A still further aspect of the present invention relates to an agent for inhibiting the degradation of hepatocyte nuclear factor 4α, wherein the agent inhibits the activity of m-calpain and/or µ-calpain.

A further aspect of the present invention relates to an agent for inhibiting the degradation of hepatocyte nuclear factor 1α, wherein the agent inhibits the activity of m-calpain and/or µ-calpain.

A still further aspect of the present invention relates to an agent for inhibiting the degradation of insulin promoter factor 1, wherein the agent inhibits the activity of m-calpain and/or µ-calpain.

Another aspect of the present invention relates to a method for inhibiting production of a gene product of a glucose metabolism-related gene, wherein the method comprises degrading a transcription factor of the glucose metabolism-related gene by using calpain.

A further aspect of the present invention relates to the preceding method for inhibiting production of a gene product of a glucose metabolism-related gene, wherein calpain is m-calpain and/or µ-calpain.

A further aspect of the present invention relates to a method for inhibiting production of a gene product of a glucose metabolism-related gene, wherein the method comprises degrading at least one transcription factor selected from the group consisting of hepatocyte nuclear factor 4α, hepatocyte nuclear factor 1α, and insulin promoter factor I by using m-calpain and/or µ-calpain.

A further aspect of the present invention relates to any of the foregoing methods for inhibiting production of a gene product of a glucose metabolism-related gene, wherein the glucose metabolism-related gene is the insulin gene or glucose transporter 2 gene.

Another aspect of the present invention relates to a method for enhancing production of a gene product of a glucose metabolism-related gene, wherein the method comprises inhibiting the degradation of a transcription factor of the glucose metabolism-related gene caused by calpain.

A further aspect of the present invention relates to the preceding method for enhancing production of a gene product of a glucose metabolism-related gene, wherein calpain is m-calpain and/or µ-calpain.

A further aspect of the present invention relates to a method for enhancing production of a gene product of a glucose metabolism-related gene, wherein the method comprises inhibiting the degradation caused by m-calpain and/or µ-calpain of at least one member selected from the group consisting of hepatocyte nuclear factor 4α, hepatocyte nuclear factor 1α and insulin promoter factor 1.

A still further aspect of the present invention relates to any of the foregoing methods for enhancing production of a gene product of a glucose metabolism-related gene, wherein the glucose metabolism-related gene is the insulin gene or glucose transporter 2 gene.

Another aspect of the present invention relates to a method for regulating production of a gene product of a glucose metabolism-related gene, wherein the method comprises changing the degree of degradation of a glucose metabolism-related gene by calcium concentration.

A further aspect of the present invention relates to a method for regulating production of a gene product of a glucose metabolism-related gene, wherein the method comprises changing the degree of degradation of at least one member selected from the group consisting of hepatocyte nuclear factor 4α, hepatocyte nuclear factor 1α and insulin promoter factor 1 by calcium concentration.

A further aspect of the present invention relates to a method for enhancing production of a gene product of a glucose metabolism-related gene, wherein the method comprises using any of the foregoing methods for inhibiting the degradation of a transcription factor.

A still further aspect of the present invention relates to a method for enhancing production of a gene product of a gene on which at least one member selected from the group consisting of hepatocyte nuclear factor 4α, hepatocyte nuclear factor 1α and insulin promoter factor 1 acts as a transcription factor, wherein the method comprises using any of the foregoing methods for inhibiting the degradation of a transcription factor.

A further aspect of the present invention relates to a method for enhancing production of a gene product of the insulin gene and/or glucose transporter 2 gene, wherein the method comprises using any of the foregoing methods for inhibiting the degradation of a transcription factor.

A still further aspect of the present invention relates to a method for preventing and/or treating a disease attributable to the degradation of a transcription factor of a glucose metabolism-related gene, wherein the method comprises using any of the foregoing methods for inhibiting the degradation of a transcription factor.

A further aspect of the present invention relates to a method for preventing and/or treating a disease attributable to the degradation of at least one member selected from the group consisting of hepatocyte nuclear factor 4α, hepatocyte nuclear factor 1α and insulin promoter factor 1, wherein the method comprises using any of the foregoing methods for inhibiting the degradation of a transcription factor.

A further aspect of the present invention relates to a method for preventing and/or treating a disease attributable to a decrease in a gene product of a glucose metabolism-related gene, wherein the method comprises using any of the foregoing methods for inhibiting the degradation of a transcription factor.

A further aspect of the present invention relates to a method for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the method comprises using any of the foregoing methods for inhibiting the degradation of a transcription factor.

A further aspect of the present invention relates to a method for preventing and/or treating a disease attributable to a decrease in a gene product of the insulin gene and/or glucose transporter 2 gene, wherein the method comprises using any of the foregoing methods for inhibiting the degradation of a transcription factor.

A further aspect of the present invention relates to a method for preventing and/or treating diabetes, wherein the method comprises using any of the foregoing methods for inhibiting the degradation of a transcription factor.

A still further aspect of the present invention relates to a method for enhancing production of a gene product of a glucose metabolism-related gene, wherein the method comprises using any of the foregoing agents for inhibiting the degradation of a transcription factor.

A further aspect of the present invention relates to a method for enhancing production of a gene product of a gene on which at least one member selected from the group consisting of hepatocyte nuclear factor 4α, hepatocyte nuclear factor 1α and insulin promoter factor 1 acts as a transcription factor, wherein the method comprises using any of the foregoing agents for inhibiting the degradation of a transcription factor.

A further aspect of the present invention relates to a method for enhancing production of a gene product of the insulin gene and/or glucose transporter 2 gene, wherein the method comprises using any of the foregoing agents for inhibiting the degradation of a transcription factor.

A still further aspect of the present invention relates to a method for preventing and/or treating a disease attributable to the degradation of a transcription factor of a glucose metabolism-related gene, wherein the method comprises using any of the foregoing agents for inhibiting the degradation of a transcription factor.

A further aspect of the present invention relates to a method for preventing and/or treating a disease attributable to the degradation of at least one member selected from the group consisting of hepatocyte nuclear factor 4α, hepatocyte nuclear factor 1α and insulin promoter factor 1, wherein the method comprises using any of the foregoing agents for inhibiting the degradation of a transcription factor.

A further aspect of the present invention relates to a method for preventing and/or treating a disease attributable to a decrease in a gene product of a glucose metabolism-related gene, wherein the method comprises using any of the foregoing agents for inhibiting the degradation of a transcription factor.

A further aspect of the present invention relates to a method for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the method comprises using any of the foregoing agents for inhibiting the degradation of a transcription factor.

A still further aspect of the present invention relates to a method for preventing and/or treating a disease attributable to a decrease in a gene product of the insulin gene and/or glucose transporter 2 gene, wherein the method comprises using any of the foregoing agents for inhibiting the degradation of a transcription factor.

A further aspect of the present invention relates to a method for preventing and/or treating diabetes, wherein the method comprises using any of the foregoing agents for inhibiting the degradation of a transcription factor.

A still yet further aspect of the present invention relates to an agent for enhancing production of a gene product of a glucose metabolism-related gene, wherein the agent contains an effective dose of any of the foregoing agents for inhibiting the degradation of a transcription factor.

A further aspect of the present invention relates to an agent for enhancing production of a gene product of a gene on which at least one member selected from the group consisting of hepatocyte nuclear factor 4α, hepatocyte nuclear factor 1α and insulin promoter factor 1 acts as a transcription factor, wherein the agent contains an effective dose of any of the foregoing agents for inhibiting the degradation of a transcription factor.

A further aspect of the present invention relates to an agent for enhancing production of a gene product of the insulin gene and/or glucose transporter 2 gene, wherein the agent contains an effective dose of any of the foregoing agents for inhibiting the degradation of a transcription factor.

A still further aspect of the present invention relates to a pharmaceutical composition which contains an effective dose of any of the foregoing agents for inhibiting the degradation of a transcription factor.

A further aspect of the present invention relates to an agent for preventing and/or treating a disease attributable to the degradation of a transcription factor of a glucose metabolism-related gene, wherein the agent contains an effective dose of any of the foregoing agents for inhibiting the degradation of a transcription factor.

A further aspect of the present invention relates to an agent for preventing and/or treating a disease attributable to the degradation of at least one member selected from the group consisting of hepatocyte nuclear factor 4α, hepatocyte nuclear factor 1α and insulin promoter factor 1, wherein the agent contains an effective dose of any of the foregoing agents for inhibiting the degradation of a transcription factor.

A still further aspect of the present invention relates to an agent for preventing and/or treating a disease attributable to a decrease in a gene product of a glucose metabolism-related gene, wherein the agent contains an effective dose of any of the foregoing agents for inhibiting the degradation of a transcription factor.

A further aspect of the present invention relates to an agent for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the agent contains an effective dose of any of the foregoing agents for inhibiting the degradation of a transcription factor.

A further aspect of the present invention relates to an agent for preventing and/or treating a disease attributable to a decrease in a gene product of the insulin gene and/or glucose transporter 2 gene, wherein the agent contains an effective dose of any of the foregoing agents for inhibiting the degradation of a transcription factor.

A still further aspect of the present invention relates to an agent for preventing and/or treating diabetes, wherein the agent contains an effective dose of any of the foregoing agents for inhibiting the degradation of a transcription factor.

A further aspect of the present invention relates to a method for preventing and/or treating liver adenoma or hepatocellular carcinoma, wherein the method comprises using the foregoing method for inhibiting the degradation.

A further aspect of the present invention relates to a method for preventing and/or treating liver adenoma or hepatocellular carcinoma, wherein the method comprises using the foregoing degradation inhibitory agent.

A still further aspect of the present invention relates to an agent for preventing and/or treating liver adenoma or hepatocellular carcinoma, wherein the agent contains an effective dose of the foregoing degradation inhibitory agent.

A still yet further aspect of the present invention relates to a method for identifying a compound that inhibits the degradation by calpain of a transcription factor of a glucose metabolism-related gene, wherein the method comprises contacting calpain and/or the transcription factor with a test compound under conditions that allow the cleavage of the transcription factor by calpain; and determining whether the test compound inhibits the cleavage of the transcription factor by calpain, by introducing a system using a signal and/or a marker capable of detecting the degradation of the transcription factor by calpain and detecting the presence, absence or change of the signal and/or the marker.

A further aspect of the present invention relates to a method for identifying a compound that inhibits the degradation by calpain of a transcription factor of a glucose metabolism-related gene, wherein the method comprises contacting calpain and/or the transcription factor with a test compound under conditions that allow the cleavage of the transcription factor by calpain; and determining whether the test compound inhibits the cleavage of the transcription factor by calpain, by introducing a system using a signal and/or a marker capable of detecting the amount of the transcription factor or the amount of a degradation product of the transcription factor and detecting the presence, absence or change of the signal and/or the marker.

A further aspect of the present invention relates to a method for identifying a compound that inhibits the degradation by calpain of a transcription factor of a glucose metabolism-related gene, wherein the method comprises contacting calpain and/or the transcription factor with a test compound under conditions that allow the binding of calpain to the transcription factor; and determining whether the test compound inhibits the binding of calpain to the transcription factor, by introducing a system using a signal and/or a marker capable of detecting the binding of calpain to the transcription factor and detecting the presence, absence or change of the signal and/or the marker.

A further aspect of the present invention relates to any of the foregoing identification methods, wherein calpain is m-calpain or µ-calpain.

A still further aspect of the present invention relates to any of the foregoing identification methods, wherein the transcription factor of a glucose metabolism-related gene is at least one member selected from the group consisting of hepatocyte nuclear factor 4α, hepatocyte nuclear factor 1α and insulin promoter factor 1.

A further aspect of the present invention relates to a compound identified by any of the foregoing identification methods.

A further aspect of the present invention relates to a reagent kit containing at least one member selected from the group consisting of calpain, a polynucleotide encoding calpain, and a vector containing a polynucleotide encoding calpain, and at least one member selected from the group consisting of a transcription factor of a glucose metabolism-related gene that is degraded by calpain, a polynucleotide encoding the transcription factor, and a vector containing the polynucleotide.

A further aspect of the present invention relates to a reagent kit comprising at least one member selected from the group consisting of calpain, a polynucleotide encoding calpain, and a vector containing a polynucleotide encoding calpain, and at least one member selected from the group consisting of hepatocyte nuclear factor 4α, hepatocyte nuclear factor 1α, insulin promoter factor 1, a polynucleotide encoding any of these, and a vector containing the polynucleotide.

A further aspect of the present invention relates to any of the foregoing reagent kits, wherein calpain is m-calpain or µ-calpain.

### BRIEF DESCRIPTION OF THE DRAWINGS

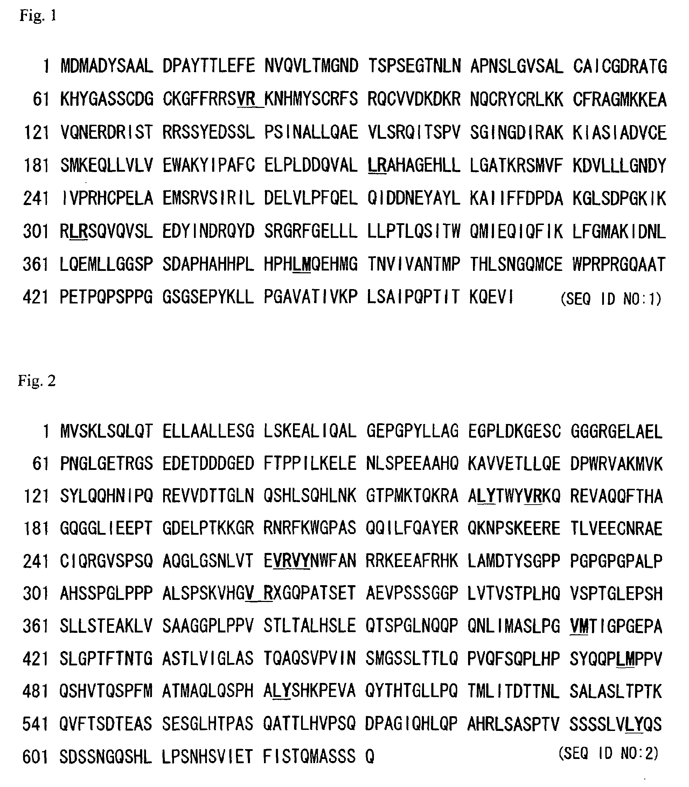

Figure 1 shows the cleavage sites in HNF-4α (SEQ ID NO: 1) that are recognized by m-calpain or µ-calpain. The amino acid sequence is represented in the single-letter code, and the recognized cleavage sites are underlined.

Figure 2 shows the cleavage sites in HNF-1α (SEQ ID NO: 2) that are recognized by m-calpain or µ-calpain. The amino acid sequence is represented in the single-letter code, and the recognized cleavage sites are underlined.

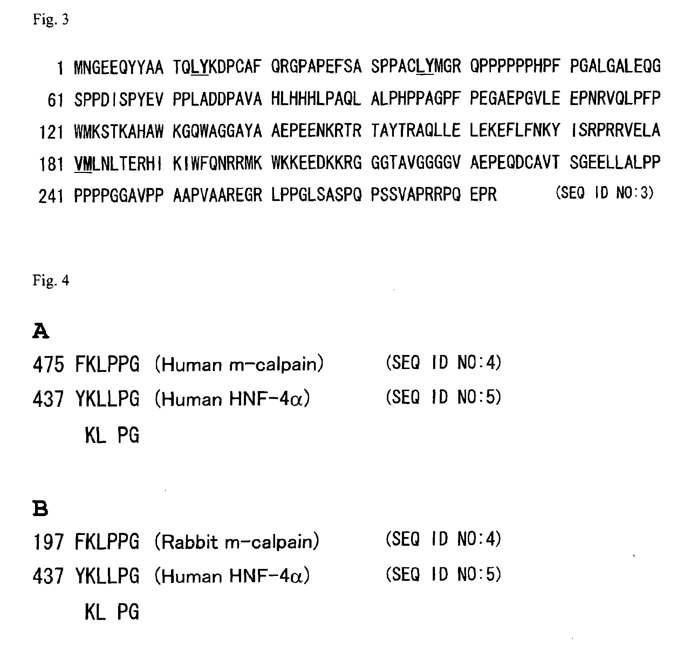

Figure 3 shows the cleavage sites in IPF-1 (SEQ ID NO: 3) that are recognized by m-calpain or µ-calpain. The amino acid sequence is represented in the single-letter code, and the recognized cleavage sites are underlined.

Figure 4 shows the results of an *in-silico* prediction of interaction of m-calpain with HNF-4α. Figure 4A shows a region that exhibited a high score as a result of local alignment between human m-calpain and human HNF-4α (SEQ ID NO: 1). Figure 4B shows a region that exhibited a high score as a result of local alignment between rabbit m-calpain and human HNF-4α (SEQ ID NO: 1). The amino acid sequences are represented in the single-letter code.

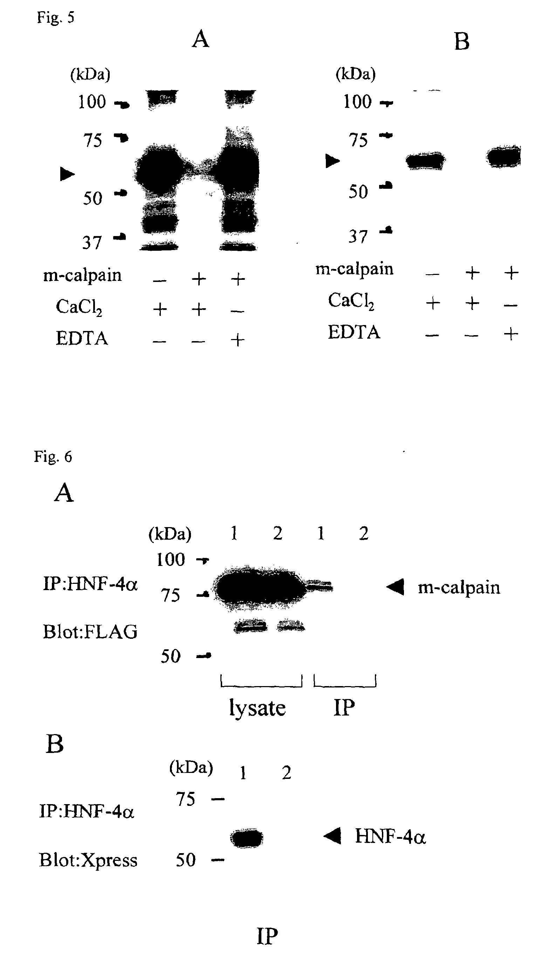

Figure 5 illustrates that rabbit m-calpain degraded human HNF-4α in the presence of calcium *in vitro*. Figure 5A and Figure 5B show the results of Western blotting using anti-HNF-4α antibody and anti-Xpress antibody, respectively. The + and - symbols in the figures indicate the presence or absence of each substance. The arrowhead indicates the band of HNF-4α. The values shown on the left-hand side of the figure represent the molecular weight of the molecular weight markers.

Figure 6 illustrates that human m-calpain bound to human HNF-4α intracellularly. Lane 1 is a sample prepared from cells in which m-calpain (FLAG-tagged) was coexpressed with HNF-4α (Xpress-tagged), and lane 2 is a sample prepared from cells in which only m-calpain (FLAG-tagged) was expressed. Figure 6A and Figure 6B show the results of Western blotting (Blot) using anti-FLAG M2 antibody and anti-Xpress antibody, respectively. In the figures, the term "IP" denotes the immunoprecipitation with anti-HNF-4α antibody. The term "lysate" denotes a sample prepared from cells, which was not immunoprecipitated. The values shown on the left-hand side of the figure represent the molecular weight of the molecular weight markers.

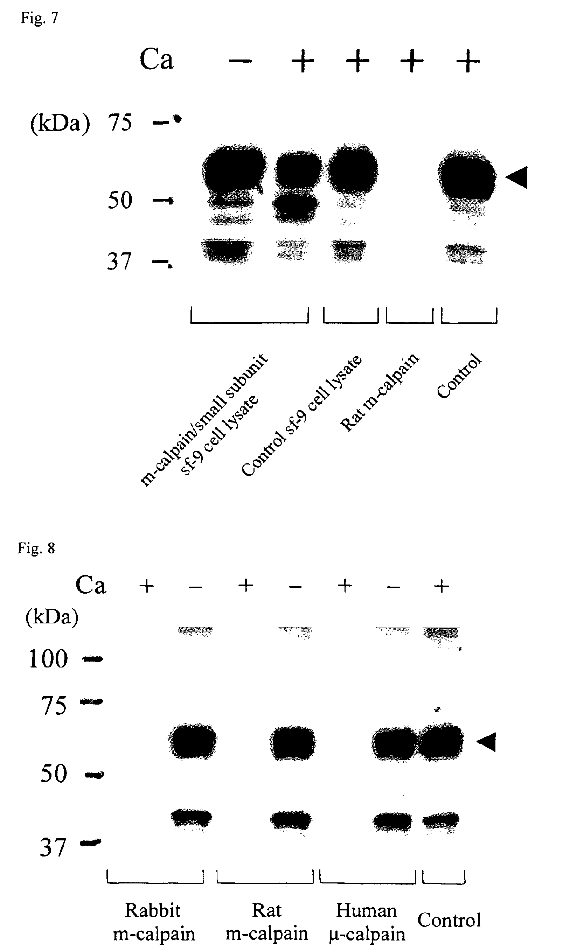

Figure 7 illustrates that human m-calpain degraded human HNF-4α in the presence of calcium *in vitro.* Lysate of insect cells that coexpressed human m-calpain with calpain small subunit 1 was used as human m-calpain. Lysate of insect cells in which calpain was not expressed was used as a negative control, as well as a sample without lysate (denoted in the figure by the term "control"). Rat m-calpain was used as a positive control. The + and - symbols in the figure indicate the presence or absence of calcium. The figure shows the results of western blotting using anti-HNF-4α antibody. The arrow head indicates the band of HNF-4α. The values shown on the left-hand side of the figure represent the molecular weight of the molecular weight markers.

Figure 8 illustrates that human µ-calpain degraded human HNF-4α in the presence of calcium *in vitro*. The + and - symbols in the figure indicate the presence or absence of calcium. Rabbit m-calpain and rat m-calpain were used as positive controls. The figure shows the results of Western blotting using anti-HNF-4α antibody. The arrowhead indicates the band of HNF-4α. The values shown on the left-hand side of the figure represent the molecular weight of the molecular weight markers.

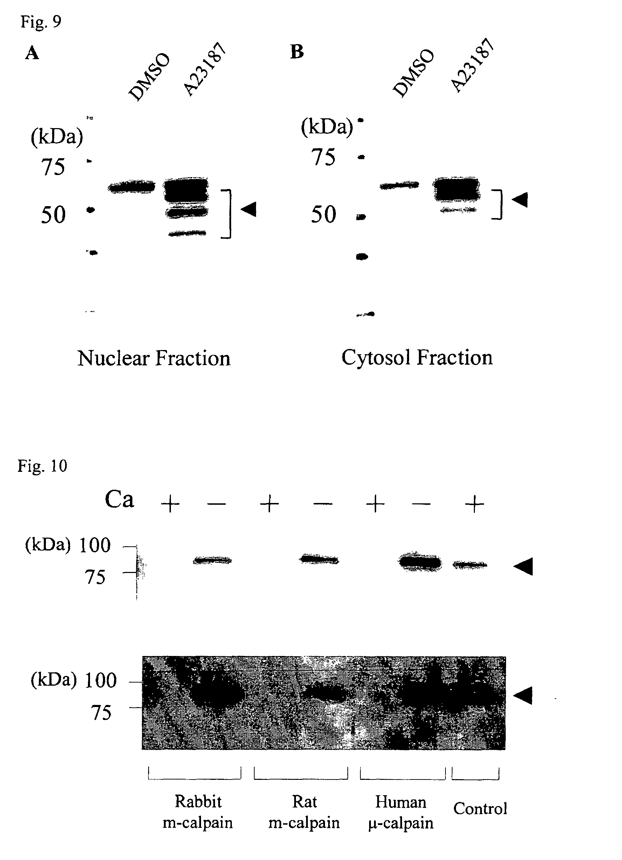

Figure 9 illustrates that HNF-4α was degraded intracellularly by the addition of an ionophore. Figure 9A and Figure 9B show the degradation of HNF-4α in a nuclear fraction and a cytoplasmic fraction, respectively. The figures show the results of Western blotting using anti-HNF-4α antibody. The arrow head indicates the band of degradation products of HNF-4α. The values shown on the left-hand side of the figure represent the molecular weight of the molecular weight markers.

Figure 10 illustrates that human µ-calpain, rabbit m-calpain and rat m-calpain degraded human HNF-1α in the presence of calcium *in vitro.* A sample without calpain was used as the control. The + and - symbols in the figure indicate the presence or absence of calcium. The upper figure and lower figure show the results of Western blotting using anti-Omni/M21 antibody and anti-HNF-1α antibody, respectively. The arrows indicate the band of HNF-1α. The values shown on the left-hand side of the figure represent the molecular weight of the molecular weight markers.

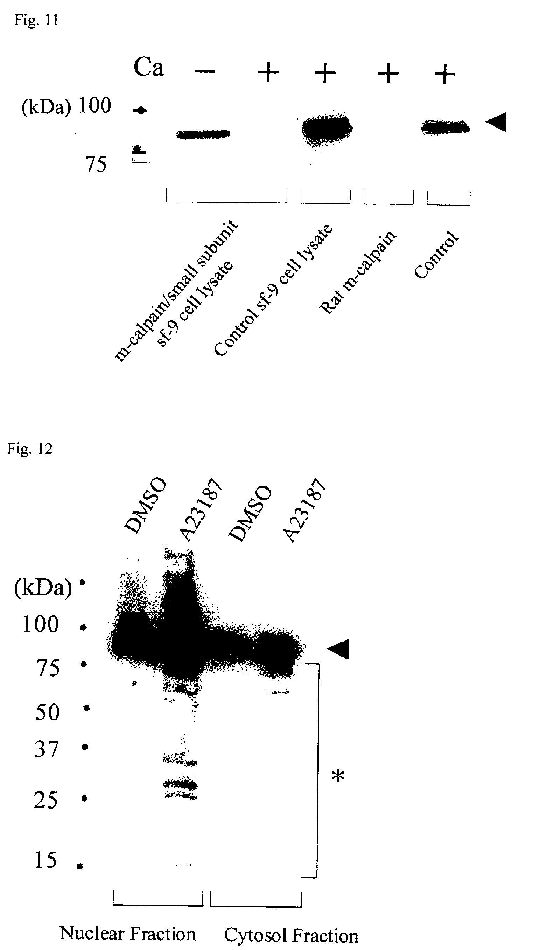

Figure 11 illustrates that human m-calpain degraded HNF-1α in the presence of calcium *in vitro.* Lysate of insect cells that coexpressed human m-calpain with calpain small subunit 1 was used as human m-calpain. Lysate of insect cells in which calpain was not expressed (denoted in the figure by the term "control sf-9 cell lysate") was used as a negative control, as well as a sample without lysate (denoted in the figure by the term "control"). Rat m-calpain was used as a positive control. The + and - symbols in the figure indicate the presence or absence of calcium. The figure shows the results of Western blotting using anti-HNF-1α antibody. The arrowhead indicates the band of HNF-1α. The values shown on the left-hand side of the figure represent the molecular weight of the molecular weight markers.

Figure 12 illustrates that HNF-1α was degraded intracellularly by the addition of an ionophore. The figure shows the results of Western blotting using anti-HNF-1α antibody. The asterisk (*) indicates the bands of degradation products of HNF-1α. The values shown on the left-hand side of the figure represent the molecular weight of the molecular weight markers.

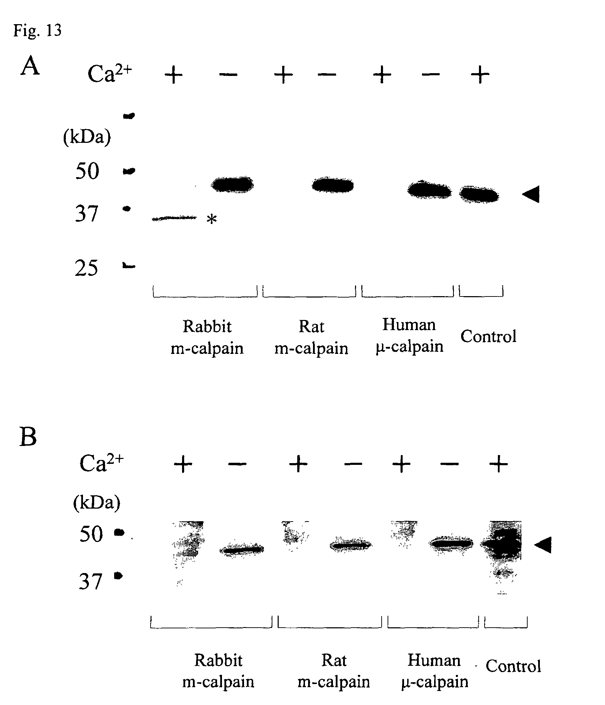

Figure 13 illustrates that human µ-calpain, rabbit m-calpain and rat m-calpain degraded human IPF-1 in the presence of calcium *in vitro.* A sample without calpain was used as a control. The + and - symbols in the figures indicate the presence or absence of calcium. Figure 13A and Figure 13B show the results of Western blotting using anti-Xpress antibody and anti-IPF-1 antibody, respectively. The arrowhead and asterisk (*) respectively indicate the band of IPF-1 and the bands of degradation products of IPF-1 produced by calpain. The values shown on the left-hand side of the figure represent the molecular weight of the molecular weight markers.

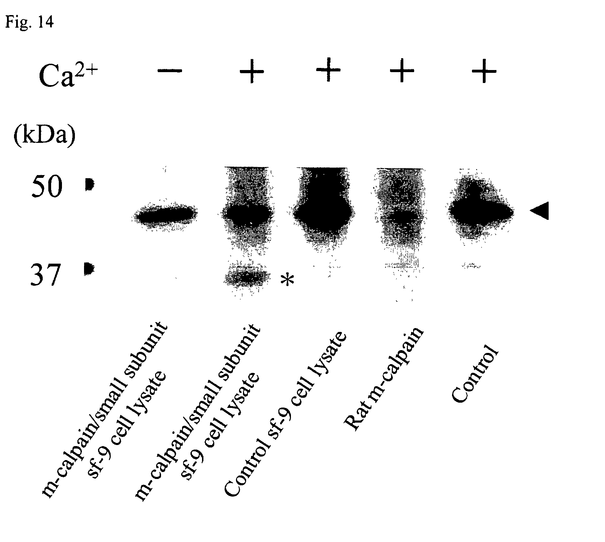

Figure 14 illustrates that human m-calpain degraded human IPF-1 in the presence of calcium *in vitro*. Lysate of insect cells that coexpressed human m-calpain with calpain small subunit 1 was used as human m-calpain. Lysate of insect cells in which calpain was not expressed (denoted in the figure by the term "control sf-9 cell lysate") was used as a negative control, as well as a sample without lysate (denoted in the figure by the term "control"). Rat m-calpain was used as a positive control. The + and - symbols in the figure indicate the presence or absence of calcium. The figure shows the results of Western blotting using anti-IPF-1 antibody. The arrowhead and asterisk (*) respectively indicate the band of IPF-1 and the bands of degradation products of IPF-1 produced by calpain. The values shown on the left-hand side of the figure represent the molecular weight of the molecular weight markers.

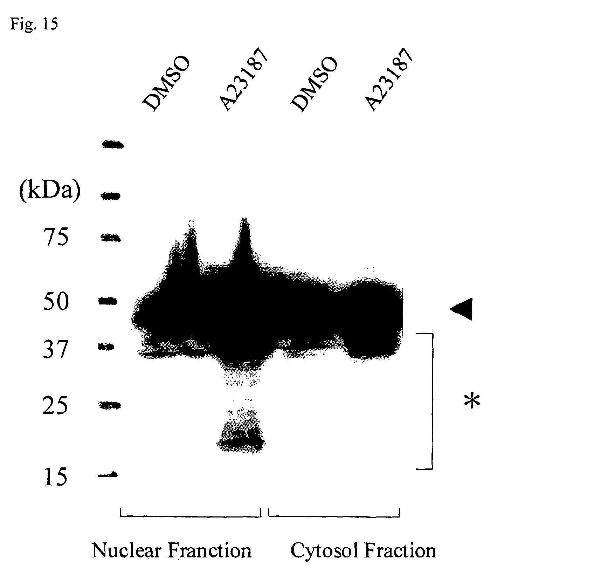

Figure 15 illustrates that IPF-1 was degraded intracellularly by the addition of an ionophore. The figure shows the results of Western blotting using anti-IPF-1 antibody. The asterisk (*) indicates the bands of degradation products of IPF-1. The values shown on the left-hand side of the figure represent the molecular weight of the molecular weight markers.

### DETAILED DESCRIPTION OF THE INVENTION

The present invention claims the benefit of priority from Japanese Patent Application Nos. 2002-254973, 2003-96370, 2003-96371 and 2003-96372, which are incorporated herein by reference.

Technical and scientific terms used herein have the meanings as normally understood by those skilled in the art, unless otherwise defined. Various methods that are well known to those skilled in the art are referenced herein. These reference materials, such as published materials disclosing known methods cited herein, are incorporated herein by reference in their entirety.

Embodiments of the present invention are explained in further detail below. However, the detailed description below is exemplary and for the purpose of explanation only, and is not intended to limit the scope of the present invention.

In the present invention, the interaction of m-calpain with HNF-4α was predicted *in-silico* according to the method described in Patent Reference 1. Further, it was revealed by way of experiment, for the first time, that m-calpain binds to HNF-4α and degrades it. In addition, it was found that µ-calpain, which belongs to the calpain super family along with m-calpain, also degrades HNF-4α. Since m-calpain binds to HNF-4α to degrade it, and µ-calpain also degrades HNF-4α, it is believed that µ-calpain also binds to HNF-4α.

It was also found that HNF-1α and IPF-1, which act as transcription factors of glucose metabolism-related genes, are also degraded by both m-calpain and µ-calpain, as is HNF-4α.

Based on amino acid motifs of the calpain-recognized cleavage sites, it was inferred that HNF-4α (SEQ ID NO: 1) is cleaved by m-calpain or µ-calpain at the following four recognized cleavage sites (Figure 1): between the arginine (R) that comes after valine (V) at position 79 in the amino acid sequence and the following lysine (K); between the arginine (R) that comes after leucine (L) at position 211 and the following alanine (A); between the arginine (R) that comes after leucine (L) at position 302 and the following serine (S); and between the methionine (M) that comes after leucine (L) at position 384 and the following glutamine (Q).

It was inferred that HNF-1α (SEQ ID NO: 2) is cleaved by m-calpain or µ-calpain at the following nine recognized cleavage sites (Figure 2): between the tyrosine (Y) that comes after leucine (L) at position 162 in the amino acid sequence and the following threonine (T); between the arginine (R) that comes after valine (V) at position 167 and the following lysine (K); between the arginine (R) that comes after valine (V) at position 262 and the following valine (V); between the tyrosine (Y) that comes after valine (V) at position 264 and the following asparagine (N); between the arginine (R) that comes after valine (V) at position 320 and the following amino acid residue (although still uncertain, it is reported to be tyrosine); between the methionine (M) that comes after valine (V) at position 411 and the following threonine (T); between the methionine (M) that comes after leucine (L) at position 476 and the following proline (P); between the tyrosine (Y) that comes after leucine (L) at position 502 and the following serine (S); and between the tyrosine (Y) that comes after leucine (L) at position 597 and the following glutamine (Q).

It was inferred that IPF-1 (SEQ ID NO: 3) is cleaved by m-calpain or µ-calpain at the following three recognized cleavage sites (Figure 3): between the tyrosine (Y) that comes after leucine (L) at position 13 in the amino acid sequence and the following lysine (K); between the tyrosine (Y) that comes after leucine (L) at position 36 and the following methionine (M); and between the methionine (M) that comes after valine (V) at position 181 and the following leucine (L).

In the present specification, an amino acid may be represented by a single letter or by three letters. Further, the term "peptide" refers to an arbitrary peptide comprising two or more amino acids that are bound to each other by a peptide bond or modified peptide bond, and the term includes a short-chain peptide such as an isolated or a synthetic full length oligopeptide, which are also referred to as an "oligomer", as well as a long-chain peptide such as an isolated or a synthetic full length polypeptide and an isolated or a synthetic full length protein.

Thus, the present invention has clarified that HNF-4α, HNF-1α and IPF-1, which have functions as a transcription factor, are degraded by calpain. HNF-4α regulates the expression of various genes encoding for substances participating in various functions such as metabolism of cholesterol, fatty acids and glucose as well as blood coagulation. HNF-1α regulates the expression of many liver specific genes such as the albumin gene, fibrinogen gene, and α1-antitrypsin gene. Further, it is reported that IPF-1 regulates the expression of glucose metabolism-related genes such as the GLUT2 gene, insulin gene, and glucokinase gene. A decrease in a transcription factor due to the degradation causes a decrease in expression of a gene on which the transcription factor acts, resulting in an abnormality of biological function which leads to a disease attributable to a decrease in the gene product of the gene. Accordingly, it is possible to prevent and treat diseases that are attributable to decreases in gene products of genes on which these transcription factors respectively act, by inhibiting the degradation of the transcription factors caused by calpain.

It is known that according to one of their functions, HNF-4α, HNF-1α and IPF-1 form transcription factor networks in pancreatic β cells and participate in the expression of glucose metabolism-related genes. The term "glucose metabolism-related genes" refers to genes encoding substances that regulate glucose metabolism (such as glucose absorption, glucose transport and glycolysis) in an organism. Examples of such substances include insulin, glucokinase and GLUT2. A decrease or a functional defect in a transcription factor involved in expression of these glucose metabolism-related genes is considered to lead to failure of transcription factor networks in pancreatic β cells resulting in an abnormal glucose metabolism, which causes a disease such as diabetes.

Degradation of a transcription factor of a glucose metabolism-related gene may be a cause of a decrease or a functional defect in the transcription factor. By inhibiting the degradation of a transcription factor of a glucose metabolism-related gene, it is possible to prevent and/or treat a disease that is attributable to a decrease or a functional defect in the transcription factor or a disease attributable to a decrease in the gene product of a gene on which the transcription factor acts.

One aspect of the present invention that was achieved based on these findings relates to a method for degrading a transcription factor of a glucose metabolism-related gene and an agent for degrading the same, wherein the method and agent comprises using calpain.

A transcription factor involved in the expression of a glucose metabolism-related gene can be exemplified by HNF-4α, HNF-1α and IPF-1. A transcription factor of a glucose metabolism-related gene that can be applied to the degradation method of the present invention is not limited to these specific examples, and any transcription factor can be applied thereto as long as it is degraded by calpain. Detection of the degradation by calpain can be conducted by bringing calpain into contact with a transcription factor in the presence of calcium and using a known method such as Western blotting.

The term "calpain" as used herein includes all proteases belonging to the calpain super family. Examples of proteases belonging to the calpain super family include, but are not limited to, m-calpain, µ-calpain, calpain 3, calpain 5, calpain 8, calpain 9, calpain 10, calpain 11, calpain 12 and calpain 13. m-Calpain or µ-calpain is preferable. µ-Calpain, which has a low calcium requirement in comparison to m-calpain, is activated by a lower calcium concentration. Therefore, µ-calpain is more easily activated by an increase in calcium concentration than m-calpain and is thought to mainly act *in vivo.* Accordingly, µ-calpain is more preferable. When using these proteases, they may be used independently, or two or more of them may be used in combination. Calpain is not limited to one that specifically degrades one kind of protein, and may be one that is involved in the degradation of more than one kind of protein, for example, more than one kind of transcription factor.

The agent for degrading a transcription factor of a glucose metabolism-related gene according to the present invention contains an effective dose of calpain as an active ingredient.

The method for degrading a transcription factor of a glucose metabolism-related gene according to the present invention comprises making calpain coexist with the transcription factor.

Since calpain is a calcium-dependent protease, it is preferable to make calpain coexist with the transcription factor of the glucose metabolism-related gene in the presence of calcium. The calcium concentration is determined with consideration to the calcium requirement of calpain. A concentration is used that can activate calpain and induce the enzyme activity thereof. For example, a suitable calcium concentration to activate m-calpain is preferably approximately 1 mM or more. A suitable calcium concentration to activate µ-calpain is preferably approximately 10 µM or more, more preferably approximately 20 µM or more, and further preferably approximately 30 µM or more. Further, since the degradation of HNF-4α, HNF-1α and IPF-1 by calpain has been revealed to be calcium dependent, it is possible to regulate the degradation of these transcription factors of glucose metabolism-related genes caused by calpain to a desired level by modulating the calcium concentration. A method for degrading a transcription factor of a glucose metabolism-related gene, which comprises modulating the calcium concentration, is also included in the scope of the present invention. The present invention also enables the construction of a degradation system for the transcription factor(s) and the utilization thereof, wherein the system includes at least one of the transcription factors and calpain, and allows calpain to coexist with the transcription factor(s).

The degradation method and degradation system for transcription factors of glucose metabolism-related genes may be *in vitro* or *in vivo.* More specifically, a degradation method and degradation system can be exemplified by those in which calpain is allowed to coexist with a transcription factor in the presence of calcium in a test tube or in a multi-well plate, for example. Alternatively, a degradation method and degradation system can be exemplified by those in which cells that are made to coexpress calpain with a transcription factor are used. A cell that is commonly used to express a protein can be used as a cell for the expression. Expression of these proteins can be carried out using known genetic engineering techniques. In a degradation method or degradation system that uses the cells, an ionophore is preferably used to modulate the intracellular calcium concentration, for example, to modulate it to a level capable of activating calpain. A known ionophore, such as A23187, can be used as the ionophore. Further, a degradation method and degradation system for the transcription factors in a non-human animal can be provided by using known genetic engineering techniques to introduce various genes encoding transcription factors of glucose metabolism-related genes and genes encoding calpain in a non-human animal.

Calpain and the transcription factor of a glucose metabolism-related gene used in the present invention can be contained in cells in which one or more of these are expressed by means of genetic engineering techniques, or can be the products of cell-free synthesis systems or the chemical synthesis products, or can be those obtained from cells or from any biological samples. These can be subsequently further purified for use. These proteins can be labeled by ligating a different type of protein or peptide thereto at the N-terminus or the C-terminus, directly or indirectly via a linker peptide and the like, by means of, for example, genetic engineering techniques. Preferably, labeling is employed that does not inhibit the interaction of calpain with the transcription factor of a glucose metabolism-related gene or the functions of these proteins, for example, the contact of calpain with the transcription factor, the enzyme activity of calpain, and the functions of the transcription factor. Examples of a labeling substance include, but are not limited to, enzymes (such as glutathione S-transferase, horseradish peroxidase, alkaline phosphatase or β-galactosidase), tag peptides (such as His-tag, Myc-tag, HA-tag, FLAG-tag, or Xpress-tag), fluorescent proteins (such as green fluorescent protein, fluorescein isothiocyanate or phycoerythrin), maltose binding protein, Fc fragment of immunoglobulin, and biotin. Alternatively, labeling may be conducted using a radioisotope. At the time of labeling, a labeling substance can be ligated alone or in combination. Detection of the labeling substance itself or of a function thereof makes it possible to determine the degradation of a transcription factor of a glucose metabolism-related gene by calpain. Each gene encoding calpain and a transcription factor(s) used for gene introduction can be prepared from a human cDNA library by known genetic engineering techniques. These genes can be introduced by known genetic engineering techniques into suitable expression vector DNA, such as, for example, a vector derived from a bacterial plasmid, to obtain a vector containing each of the above genes that is utilized for gene introduction. Gene introduction can be carried out using known genetic engineering techniques.

A degradation agent, degradation method and degradation system for a transcription factor of a glucose metabolism-related gene are useful for the following: research regarding transcription factor networks in which the transcription factor participates; elucidation of the functions of the transcription factor; and research at the molecular level regarding involvement of the transcription factor or calpain in a disease that is attributable to the degradation of the transcription factor, such as, for example, diabetes. Further, the degradation method and degradation system can also be used to construct a method for identifying compounds that inhibit the degradation of the transcription factor.

Another aspect of the present invention relates to an agent for inhibiting the degradation of a transcription factor of a glucose metabolism-related gene and a method for inhibiting the degradation of the same. The inhibitory agent and the inhibition method comprise inhibiting calpain activity, inhibiting the cleavage of the transcription factor by calpain, or inhibiting the binding of calpain to the transcription factor.

Inhibition of calpain activity or inhibition of the cleavage of a transcription factor of a glucose metabolism-related gene by calpain can be achieved, for example, by inhibiting the enzyme activity of calpain.

An agent for inhibiting the degradation of a transcription factor of a glucose metabolism-related gene, according to one aspect of the present invention, includes an effective dose of at least one substance that inhibits calpain activity as an active ingredient.

Further, a method for inhibiting the degradation of a transcription factor of a glucose metabolism-related gene, according to one aspect of the present invention, comprises using at least one substance that inhibits calpain activity. Examples of a target object, to which the inhibitory agent and the inhibition method of the present invention are applied, includes those that contain at least calpain and a transcription factor of a glucose metabolism-related gene, for example, an *in vitro* sample containing at least the same. Preferable examples of a transcription factor include HNF-4α, HNF-1α and IPF-1. An *in vitro* sample containing at least one of these transcription factors and calpain is a more preferable target object. Further, cells expressing at least calpain and a transcription factor of a glucose metabolism-related gene, for example, pancreatic β cells, are also included in the target objects as well as mammals carrying such cells.

Examples of substances having an inhibitory effect on calpain activity include low molecular weight compounds, antibodies, and peptides having a competitive inhibitory effect. Specific examples thereof include N-Acetyl-Leu-Leu-Met-CHO, N-Acetyl-Leu-Leu-Nle-CHO, Z-Leu-Leu-Tyr-CH₂F, Mu-Val-HPh-CH₂F, 4-ltuorophenylsulfonyl-Val-Leu-CHO, Leu-Leu-Phe-CH₂Cl, and Z-Val-Phe-CHO, which are known as calpain inhibitors. Examples of antibodies include an antibody that recognizes calpain or a transcription factor of a glucose metabolism-related gene and binds thereto, wherein the antibody inhibits the degradation of the transcription factor by calpain. A more preferable example of the antibody is an antibody that does not inhibit a function of the transcription factor, such as, for example, transcription factor activity. Antibodies can be produced by known methods for preparing an antibody using calpain or the transcription factor itself, a partial peptide derived from these, or a peptide comprising the amino acid sequence of an interaction site of these, as an antigen. Examples of low molecular weight compounds include a compound that inhibits the enzyme activity of calpain, preferably a compound that specifically inhibits the enzyme activity. The compound can be obtained, for example, by utilizing the degradation method or degradation system of the present invention to identify a compound that inhibits the degradation of a transcription factor of a glucose metabolism-related gene by calpain. The term "specifically inhibits calpain" refers to a condition where a compound strongly inhibits calpain but does not inhibit other enzymes or only slightly inhibits other enzymes.

Inhibition of the cleavage of a transcription factor of a glucose metabolism-related gene by calpain can also be achieved by inhibiting the binding of calpain to the transcription factor.

An agent for inhibiting the degradation of a transcription factor of a glucose metabolism-related gene, according to one aspect of the present invention, contains an effective dose of at least one substance that inhibits the binding of calpain to the transcription factor as an active ingredient.

Further, a method for inhibiting the degradation of a transcription factor of a glucose metabolism-related gene, according to one aspect of the present invention, comprises using at least one substance that inhibits the binding of calpain to the transcription factor.

Inhibition of the binding of calpain to the transcription factor of interest can be carried out, for example, by using a peptide that comprises the amino acid sequence of a site where both proteins interact with each other. Such a peptide can be exemplified by a peptide that contains the amino acid sequence of a site in the amino acid sequence of the transcription factor where the factor is cleaved by calpain, that is, a recognized cleavage site. For example, it is believed that amino acid sequences of the cleavage sites recognized by m-calpain or µ-calpain are LY, LM, LR, VY, VM and VR. A peptide containing the amino acid sequence of at least one of these recognized cleavage sites are preferable. It is believed that such a peptide competitively inhibits the degradation of the transcription factor by calpain. More preferably, a peptide may be exemplified that comprises three or more consecutive amino acid residues among the amino acid sequence of the transcription factor of interest, such as, for example, the amino acid sequence set forth in SEQ ID NO: 1, SEQ ID NO: 2 or SEQ ID NO: 3, and that contains the amino acid sequence of a cleavage site recognized by calpain.

Inhibition of the binding of calpain to a transcription factor of a glucose metabolism-related gene can also be achieved by using a peptide comprising the amino acid sequence of a binding site of calpain and the transcription factor. More specifically, it is believed that a peptide derived from HNF-4α, which is a substrate for m-calpain, such as, for example, the peptide YKLLPG (SEQ ID NO: 5, see Example 1 and Figure 4) or a peptide containing this peptide, competitively inhibits the interaction between the two proteins.

The aforementioned peptide can be designed based on the amino acid sequence of calpain or a transcription factor of a glucose metabolism-related gene, synthesized by a known peptide synthesis method, and obtained by selecting one that inhibits the cleavage of the transcription factor by calpain and/or the binding of calpain to the transcription factor.

A peptide having an amino acid sequence derived from the thus specified peptide, in which a mutation such as a deletion, substitution, addition or insertion of one to several amino acids was introduced, is also included in the scope of the present invention, as long as the peptide inhibits the binding of calpain to a transcription factor of a glucose metabolism-related gene. A peptide that also inhibits the cleavage of the transcription factor by calpain is preferable for such a peptide into which the mutation is introduced. A peptide having the mutation may be a naturally existing peptide or a peptide in which a mutation was introduced. Techniques for introducing a mutation such as a deletion, substitution, addition or insertion are known. For example, the Ulmer technique (Non-patent Reference 41) may be utilized. When introducing a mutation as described above, in view of avoiding a change in the fundamental properties (such as physical properties, function, physiological activity, and immunological activity) of the polypeptide, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived.

A peptide that can inhibit the binding of calpain to a transcription factor of a glucose metabolism-related gene can be modified to the extent that no significant functional change is involved, such as modification of its constituent amino group or carboxyl group or the like by an amidation or the like. In particular, a modification that is commonly used to stabilize the binding of a peptide to another protein to make the peptide less prone to dissociation therefrom, such as, for example, a modification such as formylation of the C terminus or acetylation of the N terminus, is useful for enhancing the effectiveness of a peptide that inhibits the binding of calpain to the transcription factor.

The aforementioned peptide can be produced by common methods that are known in peptide chemistry. A method described in the References (Non-patent Reference 42 and Non-patent Reference 43), for example, may be used, although the methods are not limited thereto and known methods can be broadly utilized.

An agent for inhibiting the degradation of a transcription factor of a glucose metabolism-related gene, according to one aspect of the present invention, contains as an active ingredient, an effective dose of at least one member selected from the group consisting of the following: a substance inhibiting the binding of the transcription factor to calpain; a substance inhibiting the cleavage of the transcription factor by calpain; and a substance inhibiting calpain activity. The agent for inhibiting the degradation of a transcription factor may be an inhibitory agent containing, as an active ingredient, an effective dose of at least one member selected from the group consisting of the following: a substance inhibiting the binding of one kind of transcription factor to calpain; a substance inhibiting the cleavage of the transcription factor by calpain; and a substance inhibiting calpain activity. Further, the inhibitory agent may contain a combination of plural kinds of inhibitory substances for each transcription factor. More specifically, for example, the inhibitory agent preferably contains as an active ingredient, an effective dose of at least one member selected from the group consisting of the following: a substance inhibiting the binding of calpain to HNF-4α, HNF-1α, or IPF-1α; a substance inhibiting the cleavage of HNF-4α, HNF-1α, or IPF-1α by calpain; and a substance inhibiting calpain activity.

A method for inhibiting the degradation of a transcription factor of a glucose metabolism-related gene, according to one aspect of the present invention, comprises using at least one member selected from the group consisting of the following: a substance inhibiting the binding of calpain to the transcription factor; a substance inhibiting the cleavage of the transcription factor by calpain; and a substance inhibiting calpain activity. In this case, the method for inhibiting the degradation of a transcription factor may comprise using at least one member selected from the group consisting of the following: a substance inhibiting the binding of calpain to one kind of transcription factor; a substance inhibiting the cleavage of the transcription factor by calpain; and a substance inhibiting calpain activity, as well as using plural kinds of such inhibitory substances for each transcription factor in combination. More specifically, for example, the method preferably comprises using at least one member selected from the group consisting of the following: a substance inhibiting the binding of calpain to HNF-4α, HNF-1α, or IPF-1α; a substance inhibiting the cleavage of HNF-4α, HNF-1α, or IPF-1α by calpain; and a substance inhibiting calpain activity.

A further aspect of the present invention relates to a method for regulating production of a gene product of a glucose metabolism-related gene. The method for regulating production of a gene product of a glucose metabolism-related gene, according to the present invention, comprises regulating the degradation caused by calpain of a transcription factor involved in expression of a gene encoding the gene product and thereby regulating production of the gene product of the gene.

One aspect of the method for regulating production of a gene product of a glucose metabolism-related gene is a method for enhancing production of a gene product of a glucose metabolism-related gene, which comprises inhibiting the degradation caused by calpain of a transcription factor involved in the gene expression. More specifically, the method for enhancing production can be accomplished by use of an agent for inhibiting the degradation of the transcription factor or a method for inhibiting the degradation of the same. An agent for enhancing production of a gene product of a glucose metabolism-related gene, which contains the aforementioned degradation inhibitory agent, is also included in the scope of the present invention.

Another aspect of the method for regulating production of a gene product of a glucose metabolism-related gene can be a method that comprises regulating production of a gene product of a glucose metabolism-related gene by modulating the calcium concentration to regulate the degradation of a transcription factor involved in expression of the factor to a desired degree. By providing a high calcium concentration to activate calpain, a transcription factor involved in expression of a glucose metabolism-related gene can be degraded, and thereby production of the gene product of a gene on which the transcription factor acts can be lowered. Further, by providing a low calcium concentration to attenuate the enzyme activity of calpain, the degradation of the transcription factor can be inhibited, and thereby production of the gene product can be enhanced.

The gene product of a gene on which a transcription factor involved in expression of a glucose metabolism-related gene acts can be exemplified by the gene product of a gene having a binding site for the transcription factor within its promoter or enhancer. More specifically, examples of a gene on which HNF-4α acts include a gene having an HNF-4α binding site within its promoter or enhancer, such as the insulin gene, HNF-1α gene, blood coagulation factor VII gene and blood coagulation factor IX gene. Examples of a gene on which HNF-1α acts include a gene having an HNF-1α binding site within a promoter or enhancer, such as the insulin gene, GLUT2 gene, HNF-4α gene and IPF-1 gene. Examples of a gene on which IPF-1 acts include a gene having an IPF-1 binding site within a promoter or enhancer, such as a GLUT2 gene.

A still further aspect of the present invention relates to an agent for preventing and/or treating a disease attributable to the degradation of a transcription factor involved in expression of a glucose metabolism-related gene, as well as a method for preventing and/or treating the disease. The agent for preventing and/or treating the disease contains the aforementioned degradation inhibitory agent. The method for preventing and/or treating the disease can be accomplished by using the aforementioned degradation inhibitory agent or the aforementioned degradation inhibition method.

A disease that is attributable to the degradation of a transcription factor involved in expression of a glucose metabolism-related gene can be exemplified by a disease that is attributable to a decrease in a gene product of a gene on which the transcription factor acts. For example, HNF-4α acts on the insulin gene and contributes to the production of the gene product thereof. HNF-1α acts on the insulin gene and GLUT2 gene and contributes to the production of the gene product of these genes. Further, HNF-1α and HNF-4α act as transcription factors on each other's genes, and enhance the production of their respective gene products, and thereby contribute further to the production of the gene product of the insulin gene. IPF-1 acts on the GLUT2 gene to contribute to the production of the gene product of the gene. IPF-1 also acts as a transcription factor for HNF-4α to promote the expression thereof, and thereby contributes to the production of the gene product of the insulin gene through enhancing the production of HNF-4α.

Specific examples of a disease that is attributable to the degradation of a transcription factor involved in expression of a glucose metabolism-related gene include diseases that are attributable to a decrease in the gene product of a glucose metabolism-related gene, such as a disease that is attributable to a decrease in insulin or GLUT2. More specifically, a disease attributable to an abnormal glucose metabolism, such as, for example, diabetes or the like, may be included.

In fact, it is reported that when the pancreatic islet of a mouse is exposed to a calpain inhibitor, insulin secretion in response to glucose stimulation increases (Non-patent Reference 15). However, in that report, there is no disclosure concerning the relation between calpain and transcription factors involved in expression of glucose metabolism-related genes. Further, when a state of hyperglycemia persists in the pancreatic islet, the intracellular calcium concentration increases, resulting in desensitization of glucose-induced insulin secretion (Non-patent Reference 44).

Based on the findings of the present invention and the above information, it is believed that a cascade exists in which calpain is activated by an increase in the intracellular calcium concentration resulting from long-term hyperglycemia, and as a result, transcription factors involved in expression of glucose metabolism-related genes are degraded, whereby transcription factor networks in pancreatic β cells collapse, resulting in an abnormal glucose metabolism, which induces a pathology similar to type 2 diabetes mellitus.

Therefore, the present invention makes it possible to inhibit the degradation caused by calpain of a transcription factor involved in expression of a glucose metabolism-related gene in the above cascade and therefore to prevent and/or treat a disease that is attributable to a decrease in a gene product of a glucose metabolism-related gene, such as, for example, diabetes, through the normalization of glucose metabolism.

It is known that HNF-4α also acts as a transcription factor for genes other than glucose metabolism-related genes. For example, it is known that an HNF-4α binding site is present in a promoter or enhancer site of the blood coagulation factor VII gene or factor IX gene. It is thus believed that hemorrhagic diseases attributable to a deficiency in the gene product of each of these genes, such as haemophilia or severe factor VII deficiency, may be caused by HNF-4α degradation. The degradation inhibitory agent or degradation inhibition method according to the present invention is useful for prevention and/or treatment these diseases.

It is reported that inactivation of HNF-1α is a cause of liver adenoma (Non-patent Reference 34). It is thus believed that liver adenoma, and hepatocellular carcinoma into which liver adenoma has been transformed, may be caused by HNF-1α degradation. Therefore the degradation inhibitory agent or degradation inhibition method according to the present invention is useful for prevention and/or treatment of these diseases.

A further aspect of the present invention relates to a method for identifying a compound that inhibits the degradation by calpain of transcription factors involved in the expression of glucose metabolism-related genes. The identification method can be constructed utilizing a known pharmaceutical screening system. The identification method can be implemented utilizing the degradation system or degradation method of the present invention. Examples of a test compound include a compound derived from a natural product or chemical library, as well as a compound obtained by drug design based on the primary structure or tertiary structure of calpain and a transcription factor of a glucose metabolism-related gene. Alternatively, a compound obtained by drug design based on the structure of a peptide of a recognized cleavage site of the transcription factor by calpain or the like is also suitable as a test compound.

More specifically, a compound that inhibits the degradation of a transcription factor by calpain can be identified by selecting conditions that allow for the cleavage by calpain of a transcription factor involved in expression of glucose metabolism-related genes, contacting the test compound with calpain and/or the transcription factor under the conditions, employing a system that uses a signal and/or a marker capable of detecting degradation of the transcription factor, and then detecting the presence, the absence or the change of the signal and/or the marker. Examples of conditions that allow for the cleavage of a transcription factor by calpain include a condition such as the presence of calcium in a concentration that is enough to activate calpain. The conditions may be a condition *in vitro* or *in vivo.* For example, a cell in which calpain is coexpressed with the transcription factor can also be used. Contact of the test compound with calpain and/or the transcription factor may be conducted prior to a degradation reaction of the transcription factor by calpain, or may be conducted by allowing the test compound to coexist in the degradation reaction. As used herein, the term "signal" refers to a substance that can be detected directly based on its physical properties or chemical properties, and the term "marker" refers to a substance that can be detected indirectly by using the physical properties or biological properties as an indicator. Examples of a signal that can be used herein include luciferase, green fluorescent protein and radioactive isotopes. Examples of a marker include a reporter gene such as a chloramphenicol acetyl transferase gene, or an epitope tag for detection such as 6xHis-tag. Any labeling substance may also be used as long as it is one that is commonly used in a method for identifying compounds. These signals or markers may be used independently or in combinations of two or more. Methods for detecting these signals or markers are known to those skilled in the art. As a convenient method, degradation of a transcription factor of a glucose metabolism-related gene can be detected by determining the presence, the absence and/or the change of the transcription factor or the amount of a degradation product of the transcription factor. Determination of the amount of the transcription factor or the amount of a degradation product of the transcription factor can be carried out using a known method for detecting a protein or peptide, such as, for example, Western blotting. Alternatively, detection of the degradation of the transcription factor can be carried out by determining the presence, absence or change of the transcription factor activity. More specifically, for example, when the transcription factor is one for the insulin gene, identification of a desired compound can be conducted by transfecting a plasmid, which contains a reporter gene having a promoter region of the insulin gene that is incorporated in the upstream thereof, to a cell that expresses the transcription factor and calpain, and then comparing the expression amount of the reporter gene when contacting a test compound with that cell with the expression amount of the reporter gene when not contacting the test compound with the cell. When the transcription factor is one for the GLUT2 gene, identification of a desired compound can be conducted by transfecting a plasmid, which contains a reporter gene having a promoter region of the GLUT2 gene that is incorporated in the upstream thereof, to a cell expressing the transcription factor and calpain, and then carrying out comparison in a similar manner as in the foregoing. The method is not limited to these specific examples. It is possible to identify a compound by using a reporter gene having an incorporated promoter region derived from a certain gene on which a transcription factor that is degraded by calpain acts.

In addition, a compound that inhibits the degradation by calpain of a transcription factor of a glucose metabolism-related gene can be identified by selecting conditions that allow for the binding of calpain to the transcription factor, contacting calpain and/or the transcription factor with a test compound under the conditions, employing a system that uses a signal and/or a marker capable of detecting the binding of calpain to the transcription factor, and then detecting the presence, absence or change of the signal and/or the marker. Detection of the binding of calpain to the transcription factor can be carried out using a known detection method, such as, for example, Western blotting.

A compound obtained by the identification method can be utilized as an agent for inhibiting the degradation of a transcription factor of a glucose metabolism-related gene or an agent for enhancing production of the gene product of a gene on which the transcription factor acts. The agents for inhibiting the degradation of a transcription factor or the agent for enhancing production of the gene product of a gene on which the transcription factor acts can be selected in consideration of a balance between biological usefulness and toxicity to prepare a pharmaceutical composition. The inhibitory agents and the enhancing agents can be used independently or in a combination of plural kinds thereof to prepare a pharmaceutical composition.

Preferably, the pharmaceutical composition of the present invention is prepared using one or more kinds of a pharmaceutical carrier. Examples of a pharmaceutical carrier include a diluent or excipient such as a filler, expander, binder, wetting agent, disintegrator, surfactant, or lubricant that are generally used in accordance with the form of use of the formulation, and these can be suitably selected and used in accordance with the administration route of the formulation to be obtained. For example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose may be included. One or a combination of two or more kinds of these may be suitably selected and used in accordance with the administration form of the present invention. As desired, various components that can be used in conventional protein preparation, such as, for example, a stabilizer, bacteriocide, buffer agent, isotonizing agent, chelating agent, pH adjuster or surfactant, can be suitably used in the formulation.

The pharmaceutical composition of the present invention can be used as an agent for preventing and/or treating a disease attributable to the degradation of a transcription factor involved in expression of a glucose metabolism-related gene. The pharmaceutical composition can also be used in a method for preventing and/or treating the disease.

Suitable dosage ranges of the pharmaceutical composition are not particularly limited, and can be determined according to the following: effectiveness of the ingredients contained therein; the administration form; the route of administration; the type of disease; the characteristics of the subject (e.g., body weight, age, symptomatic conditions and whether being taking other medicament) and the judgment of the physician in charge. In general, a suitable dosage may fall, for example, within a range of about 0.01 µg to 100 mg per 1 kg of the body weight of the subject, and preferably within a range of about 0.1 µg to 1 mg per 1 kg. However, a dosage may be altered using conventional experiments for optimization of a dosage that are well known in the art. The aforementioned dosage can be divided for administration once to several times a day. Alternatively, periodic administration once every few days or few weeks can be employed.

When administering the pharmaceutical composition of the present invention, the pharmaceutical composition may be used alone or may be used together with other compounds or medicaments necessary for the treatment.

In terms of a route of administration, it may be either systemic administration or local administration. The route of administration that is appropriate for a particular disease, symptomatic conditions, or other factors should be selected. As examples, parenteral administration including normal intravenous injection, intraarterial administration, subcutaneous administration, intracutaneous administration and intramuscular administration can be employed. Oral administration can be also employed. Further, transmucosal administration or dermal administration can be employed. When using the pharmaceutical composition for a neoplastic disease, direct administration to the neoplasm by an injection or the like is preferable.

In terms of an administration form, various forms can be selected in accordance with a treatment purpose, and typical examples thereof include an administration form of solid formulation such as a tablet, a pill, powder, powdered drug, fine granule, granule or a capsule, as well as an administration form of liquid formulation such as an aqueous formulation, ethanol formulation, suspension, fat emulsion, liposome formulation, clathrate such as cyclodextrin, a syrup and an elixir. These can be further classified according to the administration route into oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

Powders, pills, capsules, and tablets can be prepared using an excipient such as lactose, glucose, sucrose, or mannitol; a disintegrant agent such as starch or sodium alginate; a lubricant such as magnesium stearate or talc; a binder such as polyvinyl alcohol, hydroxypropyl cellulose, or gelatin; a surfactant such as fatty acid ester; a plasticizer such as glycerin, and the like. For preparation of a tablet or a capsule, a pharmaceutical carrier in a solid state is used.

A suspension can be prepared using water; sugars such as sucrose, sorbitol, or fructose; glycols such as PEG; and oils.

Injectable solutions can be prepared using a carrier comprising a salt solution, a glucose solution or a mixture of salt water and a glucose solution.

Inclusion into a liposome formulation can be conducted in the following manner: by dissolving the substance of interest in a solvent (e.g., ethanol) to make a solution, adding a solution of phospholipids dissolved in an organic solvent (e.g., chloroform), removing the solvent by evaporation and adding a phosphate buffer thereto, agitating the solution and then subjecting it to sonication followed by centrifugation to obtain a supernatant, and finally, filtrating the supernatant to recover liposomes.

A fat emulsion can be prepared in the following manner: by mixing the substance of interest, an oil ingredient (vegetable oil such as soybean oil, sesame oil, olive oil, or MCT), an emulsifier (such as a phospholipid), and the like; heating the mixture to make a solution; adding water of a required quantity; and then emulsifying or homogenizing by use of an emulsifier (a homogenizer, e.g., a high pressure jet type, an ultrasonic type, or the like). The fat emulsion may be also lyophilized. For conducting lipid-emulsification, an auxiliary emulsifier may be added, and examples thereof include glycerin or saccharides (e.g., glucose, sorbitol, fructose, etc.).

Inclusion into a cyclodextrin formulation may be carried out in the following manner: by dissolving the substance of interest in a solvent (e.g., ethanol); adding a solution of cyclodextrin dissolved in water under heating thereto; chilling the solution and filtering the precipitates; and drying under sterilization. At this time, the cyclodextrin to be used may be appropriately selected from among those having different void sizes (α, β, or γ type) in accordance with the bulkiness of the substance of interest.

A still further aspect of the present invention relates to a reagent kit including at least one member selected from the group consisting of calpain, a polynucleotide encoding calpain, and a vector containing a polynucleotide encoding calpain; and at least one member selected from the group consisting of a transcription factor involved in expression of a glucose metabolism-related gene, a polynucleotide encoding the transcription factor, and a vector containing the polynucleotide. Preferable examples of a transcription factor to be included in the reagent kit include HNF-4α, HNF-1α and 1PF-1. The reagent kit can include plural kinds of the transcription factors in combination. The reagent kit can be used, for example, in the identification method of the present invention.

A reagent comprising the aforementioned peptide and the compound that is obtained in the aforementioned identification method, as well as a reagent kit including the same, is also included in the scope of the present invention.

The reagent kit may include substances that are necessary for carrying out a determination, such as a signal and/or a marker for detecting the degradation of a transcription factor of a glucose metabolism-related gene by calpain, detection agents for these, reaction diluents, buffer solutions, washing agents and reaction stop solutions. The reagent kit may also include substances such as stabilizers and/or antiseptic agents. At the time of preparation, methods for preparation may be introduced in accordance with the respective substances to be used.

These reagents and reagent kit are useful, for example, for research concerning networks of transcription factors of glucose metabolism-related genes, as well as research at the molecular level concerning involvement of the transcription factors or calpain in a disease that is attributable to the degradation or the dysfunction of the transcription factors, such as, for example, diabetes.

### EXAMPLES

Hereinafter, the present invention may be explained more particularly with examples; however, the present invention is not limited to the following examples.

### Example 1

### (In-silico search for proteins interacting with m-calpain)

The prediction of proteins that interact with m-calpain was conducted according to the method described in Patent Reference 1. First, the amino acid sequence of m-calpain was decomposed into peptides having a pre-determined length in order to search in a database for proteins having the amino acid sequence of each of the peptides, or having homologous amino acid sequences to these amino acid sequences. Next, local alignment was conducted between the proteins obtained and m-calpain to identify proteins having a high local alignment score that might be capable of interacting with m-calpain.

As a result of analysis, it was found that the peptide YKLLPG (SEQ ID NO: 5), which has homology to the peptide FKLPPG (SEQ ID NO: 4) comprising 6 amino acid residues derived from m-calpain, are present in the amino acid sequence of HNF-4α, which is one of the nuclear transcription factors known to regulate expression of liver genes (Figure 4A and B).
Figure 4A shows the result of local alignment between human m-calpain and human HNF-4α, and Figure 4B shows the result of local alignment between rabbit m-calpain and human HNF-4α. Herein, amino acid sequences are represented in the single-letter code. It was thus predicted that HNF-4α is a protein that interacts with m-calpain. With respect to rabbit m-calpain, sequence information registered in the NCBI database only describes a region corresponding to a sequence following the amino acid residue at position 279 in the amino acid sequence of human m-calpain, wherein the homology of the region is 94%. It is thereby believed that the amino acid sequence preceding position 279 of rabbit m-calpain is almost equal to that of human m-calpain as well.

### Example 2

### (Degradation of human HNF-4α by rabbit m-calpain)

Degradation of HNF-4α by m-calpain was examined by *in vitro* degradation tests using rabbit m-calpain.

### <Materials>

An HNF-4α expression plasmid was constructed in the following manner. First, HNF-4α cDNA was obtained from human brain polyA⁺ RNA by reverse transcription polymerase chain reaction (RT-PCR). Base substitutions presumably resulting from PCR errors were corrected using the QuikChange Site-Directed Mutagenesis kit (Stratagene, Inc.). Thereafter, the cDNA was inserted at the EcoRI site of pcDNA 3.1/His (Invitrogen, Inc.), which is an expression plasmid for animal cells and is capable of generating a His and Xpress-tagged N-terminus, to construct the HNF-4α expression plasmid. An amino acid sequence encoded by the cloned HNF-4α cDNA was identical to accession number XP_009514 in the NCBI database (registered gene name is HNF4A). In this connection, in 2003, accession number XP_009514 was changed to accession number P41235 in the Swiss-Prot database.

Using the HNF-4α expression plasmid, *in vitro* synthesis of HNF-4α protein was conducted using the TNT T7 Quick Coupled Transcription/Translation System (Promega, Inc.). That is, the HNF-4α expression plasmid was mixed with TNT T7 Quick Master Mix, and then incubated for 1.5 hours at 30 °C in the presence of methionine to synthesize HNF-4α.

### <Methods>

The *in vitro* protein degradation test was carried out by adding m-calpain extracted from rabbit muscle (Protease, Calcium-Activated Neutral (calpain), Sigma Chemical Co.) at a final concentration of 50 µg/mL to the synthesized HNF-4α, and then incubating for 1 hour at 37 °C in the presence of 200 mM Tris-HCl (pH 7.8)/1mM dithiothreitol (DTT)/6 mM CaCl₂. To conduct a degradation test in the absence of calcium, a test sample was prepared by adding 10 mM ethylenediaminetetraacetic acid (EDTA) instead of 6 mM CaCl₂, and the sample was then incubated in a similar manner. The samples after incubation were added to an equal volume of 2x sodium dodecyl sulfate (SDS) sample buffer (4% SDS/125 mM Tris-HCl, pH 6.8/20% glycerol/0.01% bromphenol blue (BPB)/10% β-mercaptoethanol), heated for 5 minutes, and subjected to 10% SDS-PAGE for separation. Thereafter, Western blotting was carried out to detect HNF-4α using anti-HNF-4α antibody/HNF-4α (C-19) antibody (Santa Cruz Biotechnology, Inc.) and anti-Xpress antibody (Invitrogen, Inc.). An ECL Western blotting detection kit (Amersham pharmacia biotech) was used for the detection.

### <Results>

As shown in Figure 5A and B, the degradation of HNF-4α by rabbit m-calpain was observed. In contrast, HNF-4α was not degraded in the absence of calcium or when m-calpain was not added. Since human m-calpain has a high homology of approximately 94% with rabbit m-calpain, it is believed that human m-calpain degrades HNF-4α in a similar manner. The above results clarified that HNF-4α is degraded by m-calpain in the presence of calcium.

### Example 3

### (Analysis of the binding of human m-calpain to human HNF-4α)

Binding of m-calpain to HNF-4α was examined by binding tests using an intracellular coexpression/immunoprecipitation method.

### <Materials>

An m-calpain expression plasmid was constructed in the following manner. First, calpain cDNA was obtained from human liver polyA⁺ RNA (Clontech, Inc.) by RT-PCR. Base substitutions presumably resulting from PCR errors were corrected using the QuikChange Site-Directed Mutagenesis kit. Thereafter, the cDNA was inserted at the Sal-I site of pCMV-Tag4 (Stratagene, Inc.), which is an expression plasmid for animal cells and is capable of generating a FLAG-tagged C-terminus, to construct the m-calpain expression plasmid. An amino acid sequence encoded by the cloned m-calpain cDNA was identical to accession number AAA35645 in the NCBI database (registered gene name is CAPN2), except for the amino acids at positions 73 and 74 where methionine-arginine (MR) is substituted by isoleucine-glutamic acid (IE). There is a conflicting description in Swiss-Prot (P17655) with respect to the substitution to IE.

The expression plasmid prepared in Example 2 was used as the human HNF-4α expression plasmid in this example.

### <Methods>

First, the m-calpain gene was co-transfected with the HNF-4α gene into HEK293T cells. More specifically, after culturing a total number of 1.3 × 10⁶ HEK293T cells for 4 hours at 37 °C in the presence of 5% CO₂ (Petri dish with 60 mm diameter), 5 µg of HNF-4α expression plasmid or pcDNA 3.1/His plasmid as a negative control was transfected thereto together with 5 µg of m-calpain expression plasmid using the FuGENE 6 Transfection Reagent (Roche Applied Science). After two days culturing, the cells were washed with cold phosphate buffered saline (-) (hereunder, referred to as "PBS(-)"), suspended in 500 µl of cell lysis buffer (20 mM HEPES, pH 7.5/ 150 mM NaCl/ 1 mM EDTA/ 1% Triton X-100/ 1 mM phenylmethyl sulfonyl fluoride (PMSF)), and left to stand on ice for 20 mins. Thereafter, the cells were subjected to centrifugation at 15,000 rpm for 20 min at 4 °C to collect the supernatant for use as cell lysate. Subsequently, the collected lysate was added to 18 µl of Protein G Sepharose 4 Fast Flow (Amersham Pharmacia Biotech) that was pretreated with TBS (pH 8.0) containing 0.1% bovine serum albumin, and mixed by inversion for 2 hours at 4 °C, followed by centrifugation to collect the supernatant. 0.6 µg of anti-HNF-4α antibody/HNF-4α (C-19) antibody was added to the collected supernatant, and mixed by inversion for 1 hour at 4 °C. Then, 20 µl of Protein G Sepharose 4 Fast Flow was added thereto and mixed by inversion for an additional 1 hour at 4 °C. Subsequently, the Protein G Sepharose was collected by centrifugation, and washed 3 times with 500 µl of washing buffer (50 mM Tris-HCl, pH 7.5/ 150 mM NaCl/ 0.2% Nonidet P-40), followed by addition of 30 µl of 2× SDS sample buffer. After heating for 5 minutes, the supernatant was subjected to 10% SDS-PAGE for separation. Thereafter, Western blotting was carried out to detect binding proteins using anti-FLAG M2 antibody (Sigma) and anti-Xpress antibody. An ECL Western blotting detection kit was used for the detection.

### <Results>

As shown in Figure 6A showing the result of immunoprecipitation with anti-HNF-4α antibody (denoted by "IP" in the figure), m-calpain (FLAG-tagged) was coprecipitated only in a test sample (lane 1 in the figure) in which it was coexpressed with HNF-4α (Xpress-tagged). Coprecipitation of m-calpain was not observed in a cell in which HNF-4α was not expressed. It was thus clarified that the coprecipitation did not result from non-specific binding to agarose beads and indicates the binding of HNF-4α to m-calpain. The expression level of m-calpain was the same in both samples (see lysate in Figure 6A), which was verified from the results of Western blotting with anti-FLAG M2 antibody. Further, intracellular Xpress-HNF-4α was collected by anti-HNF-4α antibody, which was verified from the result shown in Figure 6B.

### Example 4

### (Degradation of HNF-4α by human m-calpain)

In order to investigate degradation of HNF-4α by human m-calpain, an *in vitro* degradation test was carried out using lysate of insect cells expressing human m-calpain.

### <Materials>

Human HNF-4α used in this example was prepared using a human HNF-4α expression plasmid prepared by a similar method as in Example 2.

Human m-calpain used herein was expressed in an insect cell. First, human m-calpain cDNA was prepared in a similar manner to Example 3. Further, calpain small subunit 1 cDNA was obtained from human skeletal muscle cDNA (Clontech, Inc.) by PCR, and the sequence was determined by sequencing. An amino acid sequence encoded by calpain small subunit 1 cDNA was identical to accession number NP_001740 in the NCBI database (registered gene name CAPNS1). Protein expression was conducted with an insect cell (sf-9 cell of *Spodoptera frugiperda*) expression system using baculovirus. More specifically, the respective cDNAs were inserted into pFastBac DUAL plasmid (Invitrogen, Inc.), and then Bac-to-Bac Baculovirus Expression Systems (Invitrogen, Inc.) was used to make sf-9 cells coexpress m-calpain with calpain small subunit 1. The sf-9 cells that expressed these proteins were washed with cold PBS (-) and then disrupted by freezing and thawing. Subsequently, 20 mM Tris-HCl (pH 7.5)/5 mM EDTA/10 mM β-mercaptoethanol was added thereto. After supersonic treatment on ice, the mixture was subjected to centrifugation (15,000 rpm for 30 min) to collect supernatant for use as an active sample (referred to as "cell lysate"). Further, lysate of sf-9 cells that did not express the proteins was prepared in a similar manner and used as a negative control. The presence or absence of m-calpain activity in the lysate was determined using casein as a substrate (Non-patent Reference 45). Also, rat m-calpain (Calpain 2, rat recombinant, E. coli, Calbiochem, Inc.) was used as a positive control.

### <Methods>

In-vitro protein degradation tests were carried out by adding m-calpain or rat m-calpain or the lysate of sf-9 cells that did not express calpain to HNF-4α, and incubating for 1 hour at 37 °C in the presence of 200 mM Tris-HCl (pH 7.8)/1 mM DTT/6 mM CaCl₂. The final concentration of human m-calpain in the reaction system was 2.33 mg/mL as the protein concentration of the lysate of sf-9 cells that expressed human m-calpain. The final concentration of rat m-calpain in the reaction system was 59 units/mL. A degradation test was also conducted in the same manner without addition of calpain. Further, to test for degradation in the absence of calcium, a sample was prepared by adding 10 mM of EDTA instead of 6 mM CaCl₂, and a degradation test was conducted with the sample in a similar manner. The samples after incubation were added to an equal volume of 2x SDS sample buffer, heated for 5 minutes, and subjected to 5-20% SDS-PAGE for separation. Thereafter, Western blotting was carried out to detect HNF-4α using anti-HNF-4α antibody/HNF-4α (C-19) antibody. An ECL Western blotting detection kit was used for the detection.

### <Results>

As shown in Figure 7, the degradation of human HNF-4α by human m-calpain was observed. In contrast, the degradation of HNF-4α was not observed in the absence of calcium, or in the case where the lysate of sf-9 cells that did not express calpain or the sample to which calpain was not added was used. It was thus clarified that HNF-4α is degraded by human m-calpain in the presence of calcium.

### Example 5

### (Degradation of HNF-4α by human µ-calpain)

An examination of HNF-4α degradation by human µ-calpain was carried out *in vitro* using human µ-calpain.

### <Materials>

HNF-4α protein used in this example was prepared using a human HNF-4α expression plasmid by a similar method to Example 2.

µ-Calpain extracted from human erythrocytes (Calpain 1, human erythrocytes; Calbiochem, Inc.) was used herein.

### <Methods>

*An in vitro* protein degradation test was carried out by adding µ-calpain to HNF-4α at a final concentration of 50 units/mL, and incubating for 1 hour at 37 °C in the presence of 200 mM Tris-HCl (pH 7.8)/1 mM DTT/6 mM CaCl₂. For control experiments, rabbit m-calpain (see Example 2) was added to HNF-4α at a final protein concentration of 50 µg/mL, or rat m-calpain (see Example 4) was added to HNF-4α at a final concentration of 59 units/mL, which was followed by incubation under the same conditions as above. To test for degradation in the absence of calcium, a sample was prepared by adding 10 mM of EDTA instead of 6 mM CaCl₂, and a degradation test was conducted with the sample in a similar manner. The samples after incubation were added to an equal volume of 2x SDS sample buffer, heated for 5 minutes, and subjected to 5-20% SDS-PAGE for separation. Detection of HNF-4α was conducted by the same method as in Example 4.

### <Results>

As shown in Figure 8, the degradation of HNF-4α by human µ-calpain was observed. In contrast, the degradation of HNF-4α was not observed in the absence of calcium, or in the case where the sample to which µ-calpain was not added was used. It was thus clarified that HNF-4α is degraded by µ-calpain in the presence of calcium.

### Example 6

### (Degradation of human HNF-4α by addition of ionophore)

In order to investigate intracellular degradation of HNF-4α by calpain, a test of HNF-4α degradation was conducted by adding a calcium ionophore.

### <Methods>

After culturing a total number of 0.7 × 10⁶ HEK293T cells for 22 hours at 37 °C under the presence of 5% CO₂ (Petri dish with 60 mm diameter), 2 µg of HNF-4α expression plasmid (see Example 2) was transfected thereto using the FuGENE 6 Transfection Reagent. After two days of culturing, the culture medium was replaced with a medium that contained 10 µg/mL of ionophore A23187 (4-bromo-calcium ionophore A23187, Sigma Chemical Co.), and the culturing was continued for 3 hours. For a negative control group, the culture medium was replaced with a medium to which dimethylsulfoxide (DMSO) was added instead of the ionophore. After culturing for the pre-determined time, the cells were washed with cold PBS (-), suspended in 300 µl of hypotonic cell lysis buffer (10 mM HEPES, pH 7.5/10 mM MgCl₂/42 mM KCl/1 mM PMSF), and left to stand on ice for 20 mins. The cells were disrupted with a homogenizer, and then centrifuged at 4 °C for 10 minutes at 600 g, to collect the resulting supernatant as a cytoplasmic fraction and the precipitation as a nuclear fraction. The nuclear fraction was further suspended in solution comprising 2× PBS/1% Nonidet P-40/0.1% SDS, and disrupted by ultrasonication. An equal volume of 2x SDS sample buffer was added thereto, heated for 5 minutes, and then subjected to 5-20% SDS-PAGE for separation. Detection of HNF-4α was conducted by the same method as in Example 4.

### <Results>

As shown in Figure 9A and B, the degradation of HNF-4α by adding an ionophore was observed. Based on this result, it is shown that in accordance with the increase in the intracellular concentration of calcium, HNF-4α was degraded by calpain, a calcium-dependent cysteine protease.

### Example 7

### (Degradation of human HNF-1α by calpain)

The degradation of HNF-1α by m-calpain and by µ-calpain was investigated by means of an *in vitro* protein degradation test.

### <Materials>

Human-derived µ-calpain was used in this example (see Example 5). Rabbit-derived m-calpain (see Example 2) and rat-derived m-calpain (see Example 4) were used herein.

Human HNF-1α was obtained by constructing an HNF-1α expression plasmid in the manner described hereunder and conducting protein synthesis using the plasmid. First, human HNF-1α cDNA was obtained from human liver polyA⁺ RNA by reverse transcription polymerase chain reaction (RT-PCR), and the nucleotide sequence thereof was determined by sequencing. The cDNA was then inserted at the EcoRI and NotI site of pcDNA 3.1/His, which is an expression plasmid for animal cells and is capable of generating a His and Xpress-tagged N-terminus, to construct the HNF-1α expression plasmid. An amino acid sequence encoded by the cloned HNF-1α cDNA was identical to accession number NP_000536 in the NCBI database (registered gene name is TCF1).

Using the HNF-1α expression plasmid, *in vitro* synthesis of HNF-1α protein was carried out using the TNT T7 Quick Coupled Transcription/Translation System. That is, the HNF-1α expression plasmid was mixed with TNT T7 Quick Master Mix, and then incubated for 1.5 hours at 30 °C in the presence of methionine to synthesize HNF-1α. Thereafter, immunoprecipitation was conducted by adding anti-Xpress antibody to the synthesized HNF-1α, mixing by inversion for 1 hour at 4 °C, and then adding Protein G Sepharose 4 Fast Flow and mixing by inversion for an additional 1 hour at 4 °C. Subsequently, Protein G Sepharose was collected by centrifugation and washed 3 times with washing buffer (50 mM Tris-HCl, pH 7.5/150 mM NaCl/ 0.2% Nonidet P-40), followed by rinsing with 200 mM Tris-HCl (pH 7.8) to obtain the HNF-1α sample as 20% slurry (hereunder, referred to as "HNF-1α slurry").

### <Methods>

Protein degradation tests were carried out *in vitro* by adding µ-calpain or m-calpain to the HNF-1α slurry, and incubating for 1 hour at 37 °C in the presence of 200 mM Tris-HCl (pH 7.8)/1 mM DTT/6 mM CaCl₂. The final concentrations of the respective calpain in the reaction system were as follows: 50 units/mL of human µ-calpain; 50 µg/mL of rabbit m-calpain as a protein concentration; and 59 units/mL of rat m-calpain. For a control experiment, an HNF-1α slurry was incubated without the addition of calpain in a similar manner in the presence of calcium. Further, to test for protein degradation in the absence of calcium, a sample was prepared by adding 10 mM of EDTA instead of 6 mM of CaCl₂ and was incubated in the same manner. The samples after incubation were added to an equal volume of 2x SDS sample buffer, heated for 5 minutes, and then subjected to 5-20% SDS-PAGE for separation. Thereafter, Western blotting was carried out to detect HNF-1α using anti-Omni/M21 antibody (Santa Cruz Biotechnology, Inc.), which recognizes a His-Xpress region, and anti-HNF-1α antibody/HNF-1α (C-19) antibody (Santa Cruz Biotechnology, Inc.). An ECL Western blotting detection kit was used for conducting the detection.

### <Results>

As shown in Figure 10, the *in vitro* degradation of HNF-1α by each of human µ-calpain, rabbit m-calpain and rat m-calpain was observed. In contrast, HNF-1α was not degraded in the absence of calcium, or in the case where the sample to which m-calpain was not added was used. It was thus clarified that HNF-1α is degraded by µ-calpain and m-calpain in the presence of calcium.

### Example 8

### (Degradation of HNF-1α by human m-calpain)

In order to investigate degradation of human HNF-1α by human m-calpain, *in vitro* protein degradation tests were conducted using human m-calpain expressed in an insect cell.

### <Materials>

Human HNF-1α that was prepared in Example 7 (HNF-1α slurry) was used in this example.

Human m-calpain was expressed with the sf-9 insect cell expression system by the method set forth in Example 4, and the lysate of the insect cell was used as human m-calpain in this example. In addition, lysate of sf-9 cells that did not express the protein was prepared in a similar manner and used as a negative control. The presence or absence of the m-calpain activity in the lysate was determined using casein as a substrate (Non-patent Reference 45). In addition, rat m-calpain was used as a positive control.

### <Methods>

In-vitro protein degradation tests were carried out in the same manner as in Example 7, but the lysate of sf-9 cells that coexpressed m-calpain with calpain small subunit I was used as m-calpain. The final concentration of the lysate of the sf-9 cells that expressed these proteins in the reaction system was 1.75 mg/mL as a protein concentration. The final concentration of rat m-calpain in the reaction system was 59 units/mL. Detection of HNF-1α was performed in the same manner as Example 7, but only anti-HNF-1α antibody/ HNF-1α (C-19) antibody was used.

### <Results>

As shown in Figure 11, the degradation of human HNF-1α by human m-calpain was observed. In contrast, the degradation of HNF-1α was not observed in the absence of calcium, or in the case where the lysate of sf-9 cells that did not express the protein (denoted by "control sf-9 cell lysate" in the figure) or the sample to which calpain was not added (denoted by "control" in the figure) was used. It was thus clarified that HNF-1α is degraded by human m-calpain in the presence of calcium.

### Example 9

### (Degradation of human HNF-1α by addition of ionophore)

In order to investigate intracellular degradation of HNF-1α by calpain, a test of HNF-1α degradation was conducted by adding a calcium ionophore.

### <Methods>

After culturing a total number of 0.8 × 10⁶ HEK293T cells for 5 hours at 37 °C under the presence of 5% CO₂ (Petri dish with 60 mm diameter), 2 µg of an HNF-1α expression plasmid (see Example 7) was transfected thereto using the FuGENE 6 Transfection Reagent. After two days of culturing, the culture medium was replaced with a medium containing 10 µg/mL of ionophore A23187 (Sigma Chemical Co.) and 0.2% DMSO, and then the culturing was continued for 4 hours. For a negative control group, the culture medium was replaced with a medium containing 0.2% DMSO. After culturing for the pre-determined time, the cells were washed with cold PBS (-), suspended in 350 µl of hypotonic cell lysis buffer, and then left to stand on ice for 20 mins. The cells were disrupted with a homogenizer, and then centrifuged at 4 °C for 10 minutes at 600 g to collect the resulting supernatant as a cytoplasmic fraction and the precipitation as a nuclear fraction. The nuclear fraction was further suspended in solution comprising 2x PBS/1% Nonidet P-40/0.1% SDS, and disrupted by ultrasonication. The protein concentration of cell fractions was determined with Coomassie Plus Protein Assay Reagent Kit (Pierce Chemical Corp.). Each of the prepared samples was added to an equal volume of 2x SDS sample buffer, heated for 5 minutes, and then subjected to 5-20% SDS-PAGE for separation. Detection of HNF-1α was conducted by the same method as in Example 8.

### <Results>

As shown in Figure 12, the degradation of HNF-1α by addition of the ionophore was observed. It is thus shown that in accordance with the increase in the intracellular concentration of calcium, HNF-1α was degraded by calpain, a calcium-dependent cysteine protease.

### Example 10

### (Degradation of human IPF-1 by calpain)

Degradation of IPF-1 by m-calpain and by µ-calpain was investigated by *in vitro* protein degradation tests.

### <Materials>

Human-derived µ-calpain (see Example 5) was used in this example. Rabbit-derived m-calpain (see Example 2) and rat-derived m-calpain (see Example 4) were used herein.

Human IPF-1 was obtained by constructing an IPF-1 expression plasmid in the manner described hereunder, and conducting protein synthesis using the plasmid. First, human IPF-1 cDNA was obtained from human liver polyA⁺ RNA by RT-PCR, and the nucleotide sequence thereof was determined by sequencing. Thereafter, the cDNA was inserted at BamHI and EcoRI site of pcDNA 3.1/His, which is an expression plasmid for animal cells and is capable of generating a His and Xpress-tagged N-terminus, to construct the IPF-1 expression plasmid. An amino acid sequence encoded by the cloned IPF-1 cDNA was identical to accession number NP_000200 in the NCBI database (registered gene name is IPF).

Using the IPF-1 expression plasmid, synthesis of IPF-1 protein was carried out *in vitro* using the TNT T7 Quick Coupled Transcription/Translation System. That is, the IPF-1 expression plasmid was mixed with TNT T7 Quick Master Mix, and then incubated for 1.5 hours at 30 °C in the presence of methionine to synthesize IPF-1.

### <Methods>

Protein degradation tests were carried out *in vitro* by adding µ-calpain or m-calpain to IPF-1, and incubating for 1 hour at 37 °C in the presence of 200 mM Tris-HCl (pH 7.8)/1 mM DTT/6 mM CaCl₂. The final concentrations of each calpain in the reaction systems were as follows: 50 units/mL of human µ-calpain; 50 µg/mL of rabbit m-calpain as a protein concentration, and 59 units/mL of rat m-calpain. For a control experiment, IPF-1 was incubated without addition of calpain in the same manner in the presence of calcium. Further, to test for protein degradation in the absence of calcium, a sample was prepared by adding 10 mM of EDTA instead of 6 mM of CaCl₂, and incubated in the same manner. The samples after incubation were added to an equal volume of 2x SDS sample buffer, heated for 5 minutes, and subjected to 5-20% SDS-PAGE for separation. Thereafter, Western blotting was carried out to detect IPF-1 using anti-Xpress antibody and anti-IPF1 (PDX-1) antibody/N-18 (Santa Cruz Biotechnology, Inc.). The ECL Western blotting detection kit was used for conducting the detection.

### <Results>

As shown in Figure 13A and B, the *in vitro* degradation of IPF-1 by each of human µ-calpain, rabbit m-calpain and rat m-calpain was observed. In contrast, IPF-1 was not degraded in the absence of calcium or in the case where the sample to which m-calpain was not added was used. It was thus clarified that IPF-1 is degraded by µ-calpain and m-calpain in the presence of calcium.

### Example 11

### (Degradation of IPF-1 by human m-calpain)

In order to investigate degradation of human IPF-1 by human m-calpain, *in vitro* protein degradation tests were conducted using human m-calpain expressed in an insect cell.

### <Materials>

### Human IPF-1 prepared in Example 10 was used in this example.

Human m-calpain was expressed with the insect cell sf-9 expression system by the method described in Example 4, and the insect cell lysate was used as human m-calpain. In addition, lysate of sf-9 cells that did not express the protein was prepared in a similar manner and used as a negative control. The presence or absence of the m-calpain activity in the lysate was determined using casein as a substrate (Non-patent Reference 45). Rat m-calpain was used as a positive control.

### <Methods>

In-vitro protein degradation tests were carried out in the same manner as Example 10, but the lysate of sf-9 cells that coexpressed m-calpain with calpain small subunit 1 was used as m-calpain. The final concentration of the lysate of sf-9 cells that expressed these proteins in the reaction system was 2.33 mg/mL as a protein concentration. The final concentration of rat m-calpain in the reaction system was 59 units/mL. Detection of IPF-1 was performed in the same manner as Example 10, but only anti-IPF-1 antibody/N-18 was used.

### <Results>

As shown in Figure 14, the degradation of human IPF-1 by human m-calpain was observed. In contrast, the degradation of IPF-1 was not observed in the absence of calcium, or in the case where the lysate of sf-9 cells that did not express the protein (denoted by "control sf-9 cell lysate" in the figure) or the sample to which calpain was not added (denoted by "control" in the figure)was used. It was thus clarified that IPF-1 is degraded by human m-calpain in the presence of calcium.

### Example 12

### (Degradation of human IPF-1 by addition of ionophore)

In order to investigate intracellular degradation of IPF-1 by calpain, a test of IPF-1 degradation was conducted by adding a calcium ionophore.

### <Methods>

After culturing a total number of 0.5 × 10⁶ HEK293T cells for 24 hours at 37 °C under the presence of 5% CO₂ (Petri dish with 60 mm diameter), 2 µg of IPF-1 expression plasmid (see Example 10) was transfected thereto using the FuGENE 6 Transfection Reagent. After two days of culturing, the culture medium was replaced with a medium containing 10 µg/mL of ionophore A23187 (Sigma Chemical Co.), and the culturing was continued for further 4 hours. For a negative control group, the culture medium was replaced with a medium containing 0.2% DMSO. After culturing for the pre-determined time, the cells were washed with cold PBS (-), suspended in 350 µl of hypotonic cell lysis buffer, and then left to stand on ice for 20 mins. The cells were disrupted with a homogenizer, and then centrifuged at 4 °C for 10 minutes at 600 g to collect the supernatant as a cytoplasmic fraction and the precipitation as a nuclear fraction. The nuclear fraction was further suspended in solution comprising 2× PBS/1% Nonidet P-40/0.1% SDS, and disrupted by ultrasonication. The nuclear fraction and cell fraction were added to an equal volume of 2x SDS sample buffer, heated for 5 minutes, and then subjected to 5-20% SDS-PAGE for separation. Detection of IPF-1 was conducted by the same method as in Example 10.

### <Results>

As shown in Figure 15, the degradation of IPF-1 by addition of the ionophore was observed. It is thus shown that in accordance with the increase in the intracellular concentration of calcium, IPF-1 was degraded by calpain, a calcium-dependent cysteine protease.

### INDUSTRIAL APPLICABILITY

The present invention, based on the finding that HNF-4α, HNF-1α and IPF-1, which have functions as transcription factors, are degraded by calpain, has provided a method for degrading these transcription factors by using calpain, and a method and an agent for inhibiting the degradation. It is known that these transcription factors form transcription factor networks in pancreatic β cells and participate in the expression of glucose metabolism-related genes such as the insulin gene, GLUT2 gene, and the like, and that the genes thereof are causative genes of hereditary type 2 diabetes mellitus. It is thus believed that a decrease or functional defect in transcription factors involved in the expression of glucose metabolism-related genes, where the decrease or the defect is attributable to the degradation thereof by calpain, is a cause of diabetes. Therefore, the agent for inhibiting the degradation of the transcription factors involved in expression of glucose metabolism-related genes and/or the method for inhibiting the same, which are provided according to the present invention, makes it possible to enhance the production of the gene product of genes on which the transcription factors act, such as, for example, the insulin production of the insulin gene. It is also possible to prevent and/or treat a disease attributable to decrease in the gene products of genes on which the transcription factors act. Specifically, for example, it is possible to prevent and/or treat a disease attributable to decrease in insulin, such as, more specifically, diabetes or the like. Thus, the present invention is extremely useful for preventing and/or treating a disease attributable to excessive degradation of a transcription factor involved in the expression of a glucose metabolism-related gene.

### SEQUENCE LISTING FREE TEXT

SEQ ID NO: 4: Partial peptide of human m-calpain or rabbit m-calpain showing a high score in the local alignment between human m-calpain or rabbit m-calpain and human HNF-4alpha (SEQ ID NO: 1).

SEQ ID NO: 5: partial peptide of human HNF-4α (SEQ ID NO: 1) showing a high score in local alignment of human m-calpain or rabbit m-calpain and human HNF-4α (SEQ ID NO: 1).

## Claims

1. A method for degrading a transcription factor of a glucose metabolism-related gene, wherein the method comprises making calpain coexist with the transcription factor of the glucose metabolism-related gene in the presence of calcium.

2. A method for degrading a transcription factor of a glucose metabolism-related gene, wherein the method comprises changing the degree of the degradation of the transcription factor of the glucose metabolism-related gene by calcium concentration.

3. A method for degrading a transcription factor of a glucose metabolism-related gene, wherein the method comprises making m-calpain and/or µ-calpain coexist with the transcription factor of the glucose metabolism-related gene in the presence of calcium.

4. The degradation method according to any one of claims 1 to 3, wherein the transcription factor of the glucose metabolism-related gene is at least one member selected from the group consisting of hepatocyte nuclear factor 4α, hepatocyte nuclear factor 1α and insulin promoter factor 1.

5. A method for degrading hepatocyte nuclear factor 4α (HNF-4α), wherein the method comprises making m-calpain and/or µ-calpain coexist with HNF-4α in the presence of calcium.

6. A method for degrading hepatocyte nuclear factor 1α (HNF-1α), wherein the method comprises making m-calpain and/or µ-calpain coexist with HNF-1α in the presence of calcium.

7. A method for degrading insulin promoter factor 1 (IPF-1), wherein the method comprises making m-calpain and/or µ-calpain coexist with IPF-1 in the presence of calcium.

8. A method for inhibiting the degradation of a transcription factor of a glucose metabolism-related gene, wherein the method comprises inhibiting calpain activity.

9. A method for inhibiting the degradation of a transcription factor of a glucose metabolism-related gene, wherein the method comprises inhibiting the cleavage by calpain of the transcription factor of the glucose metabolism-related gene.

10. A method for inhibiting the degradation of a transcription factor of a glucose metabolism-related gene, wherein the method comprises inhibiting the binding of calpain to the transcription factor of the glucose metabolism-related gene.

11. A method for inhibiting the degradation of a transcription factor of a glucose metabolism-related gene, wherein the method comprises treating an *in vitro* sample containing at least calpain and the transcription factor of the glucose metabolism-related gene with a substance that inhibits calpain activity.

12. A method for inhibiting the degradation of a transcription factor of a glucose metabolism-related gene, wherein the method comprises treating a cell expressing at least calpain and the transcription factor of the glucose metabolism-related gene with a substance that inhibits calpain activity.

13. The method for inhibiting the degradation of a transcription factor of a glucose metabolism-related gene according to claim 12, wherein the cell is a cell that is carried by a mammal.

14. The method for inhibiting the degradation of a transcription factor of a glucose metabolism-related gene according to claim 13, wherein the cell that is carried by a mammal is a pancreatic β cell.

15. The method for inhibiting the degradation of a transcription factor of a glucose metabolism-related gene according to any one of claims 11 to 14, wherein the substance that inhibits calpain activity is one or more substances selected from the group consisting of an antibody that recognizes calpain, an antibody that recognizes the transcription factor of the glucose metabolism-related gene, and a calpain inhibitor.

16. The method for inhibiting the degradation of a transcription factor of a glucose metabolism-related gene according to claim 15, wherein the calpain inhibitor is N-Acetyl-Leu-Leu-Met-CHO, N-Acetyl-Leu-Leu-Nle-CHO, Z-Leu-Leu-Tyr-CH₂F, Mu-Val-HPh-CH₂F, 4-fluorophenylsulfonyl-Val-Leu-CHO, Leu-Leu-Phe-CH₂Cl or Z-Val-Phe-CHO.

17. The method for inhibiting the degradation of a transcription factor of a glucose metabolism-related gene according to any one of claims 11 to 14, wherein the substance that inhibits calpain activity is a peptide containing at least one amino acid sequence of a calpain-recognized cleavage site in the transcription factor of the glucose metabolism-related gene.

18. The method for inhibiting the degradation of a transcription factor of a glucose metabolism-related gene according to any one of claims 11 to 14, wherein the substance that inhibits calpain activity is a peptide that comprises three or more consecutive amino acid residues from the amino acid sequence set forth in any of SEQ ID NOS: 1 to 3 in the sequence listing and contains at least one amino acid sequence of a calpain-recognized cleavage site in the transcription factor of the glucose metabolism-related gene.

19. The method for inhibiting the degradation of a transcription factor of a glucose metabolism-related gene according to claim 18, wherein the calpain-recognized cleavage site in the transcription factor of the glucose metabolism-related gene is selected from the group consisting of Leu-Tyr, Leu-Met, Leu-Arg, Val-Tyr, Val-Met and Val-Arg.

20. The method for inhibiting the degradation of a transcription factor of a glucose metabolism-related gene according to any one of claims 8 to 19, wherein calpain is m-calpain and/or µ-calpain.

21. The method for inhibiting the degradation of a transcription factor of a glucose metabolism-related gene according to any one of claims 8 to 20, wherein the transcription factor of the glucose metabolism-related gene is at least one member selected from the group consisting of hepatocyte nuclear factor 4α, hepatocyte nuclear factor 1α and insulin promoter factor 1.

22. A method for inhibiting the degradation of hepatocyte nuclear factor 4α, wherein the method comprises inhibiting the activity of m-calpain and/or µ-calpain.

23. A method for inhibiting the degradation of hepatocyte nuclear factor 1α, wherein the method comprises inhibiting the activity of m-calpain and/or µ-calpain.

24. A method for inhibiting the degradation of insulin promoter factor 1, wherein the method comprises inhibiting the activity of m-calpain and/or µ-calpain.

25. An agent for degrading a transcription factor of a glucose metabolism-related gene, wherein the agent contains an effective dose of calpain as an active ingredient.

26. The agent for degrading a transcription factor of a glucose metabolism-related gene according to claim 25, wherein calpain is m-calpain and/or µ-calpain.

27. The agent for degrading a transcription factor of a glucose metabolism-related gene according to claim 25 or 26, wherein the transcription factor of the glucose metabolism-related gene is at least one member selected from the group consisting of hepatocyte nuclear factor 4α, hepatocyte nuclear factor 1α and insulin promoter factor 1.

28. An agent for degrading hepatocyte nuclear factor 4α, wherein the agent contains an effective dose of m-calpain and/or µ-calpain as an active ingredient.

29. An agent for degrading hepatocyte nuclear factor 1α, wherein the agent contains an effective dose of m-calpain and/or µ-calpain as an active ingredient.

30. An agent for degrading insulin promoter factor 1, wherein the agent contains an effective dose of m-calpain and/or µ-calpain as an active ingredient.

31. An agent for inhibiting the degradation of a transcription factor of a glucose metabolism-related gene, wherein the agent inhibits calpain activity.

32. An agent for inhibiting the degradation of a transcription factor of a glucose metabolism-related gene, wherein the agent inhibits the cleavage of the transcription factor of the glucose metabolism-related gene by calpain.

33. An agent for inhibiting the degradation of a transcription factor of a glucose metabolism-related gene, wherein the agent inhibits the binding of calpain to the transcription factor of the glucose metabolism-related gene.

34. An agent for inhibiting the degradation of a transcription factor of a glucose metabolism-related gene, which contains an effective dose of a substance that inhibits calpain activity as an active ingredient.

35. The agent for inhibiting the degradation of a transcription factor of a glucose metabolism-related gene according to claim 34, wherein the substance that inhibits calpain activity is one or more substances selected from the group consisting of an antibody that recognizes calpain, an antibody that recognizes the transcription factor of the glucose metabolism-related gene, and a calpain inhibitor.

36. The agent for inhibiting the degradation of a transcription factor of a glucose metabolism-related gene according to claim 35, wherein the calpain inhibitor is N-Acetyl-Leu-Leu-Met-CHO, N-Acetyl-Leu-Leu-Nle-CHO, Z-Leu-Leu-Tyr-CH₂F, Mu-Val-HPh-CH₂F, 4-fluorophenylsulfonyl-Val-Leu-CHO, Leu-Leu-Phe-CH₂Cl or Z-Val-Phe-CHO.

37. The agent for inhibiting the degradation of a transcription factor of a glucose metabolism-related gene according to claim 34, wherein the substance that inhibits calpain activity is a peptide containing at least one amino acid sequence of a calpain-recognized cleavage site in the transcription factor of the glucose metabolism-related gene.

38. The agent for inhibiting the degradation of a transcription factor of a glucose metabolism-related gene according to claim 34, wherein the substance that inhibits calpain activity is a peptide that comprises three or more consecutive amino acid residues from the amino acid sequence set forth in any of SEQ ID NOS: 1 to 3 in the sequence listing and contains at least one amino acid sequence of a calpain-recognized cleavage site in the transcription factor of the glucose metabolism-related gene.

39. The agent for inhibiting the degradation of a transcription factor of a glucose metabolism-related gene according to claim 38, wherein the calpain-recognized cleavage site in the transcription factor of the glucose metabolism-related gene is selected from the group consisting of Leu-Tyr, Leu-Met, Leu-Arg, Val-Tyr, Val-Met and Val-Arg.

40. The agent for inhibiting the degradation of a transcription factor of a glucose metabolism-related gene according to any one of claims 31 to 39, wherein calpain is m-calpain and/or µ-calpain.

41. The agent for inhibiting the degradation of a transcription factor of a glucose metabolism-related gene according to any one of claims 31 to 40, wherein the transcription factor of the glucose metabolism-related gene is at least one member selected from the group consisting of hepatocyte nuclear factor 4α, hepatocyte nuclear factor 1α, and insulin promoter factor 1.

42. An agent for inhibiting the degradation of hepatocyte nuclear factor 4α, wherein the agent inhibits the activity of m-calpain and/or µ-calpain.

43. An agent for inhibiting the degradation of hepatocyte nuclear factor 1α, wherein the agent inhibits the activity of m-calpain and/or µ-calpain.

44. An agent for inhibiting the degradation of insulin promoter factor 1, wherein the agent inhibits the activity of m-calpain and/or µ-calpain.

45. A method for inhibiting production of a gene product of a glucose metabolism-related gene, wherein the method comprises degrading a transcription factor of the glucose metabolism-related gene by using calpain.

46. The method for inhibiting production of a gene product of a glucose metabolism-related gene according to claim 45, wherein calpain is m-calpain and/or µ-calpain.

47. A method for inhibiting production of a gene product of a glucose metabolism-related gene, wherein the method comprises degrading at least one transcription factor selected from the group consisting of hepatocyte nuclear factor 4α, hepatocyte nuclear factor 1α, and insulin promoter factor 1 by using m-calpain and/or µ-calpain.

48. The method for inhibiting production of a gene product of a glucose metabolism-related gene according to any one of claims 45 to 47, wherein the glucose metabolism-related gene is the insulin gene or glucose transporter 2 gene.

49. A method for enhancing production of a gene product of a glucose metabolism-related gene, wherein the method comprises inhibiting the degradation of a transcription factor of the glucose metabolism-related gene caused by calpain.

50. The method for enhancing production of a gene product of a glucose metabolism-related gene according to claim 49, wherein calpain is m-calpain and/or µ-calpain.

51. A method for enhancing production of a gene product of a glucose metabolism-related gene, wherein the method comprises inhibiting the degradation caused by m-calpain and/or µ-calpain of at least one member selected from the group consisting of hepatocyte nuclear factor 4α, hepatocyte nuclear factor 1α and insulin promoter factor 1.

52. The method for enhancing production of a gene product of a glucose metabolism-related gene according to any one of claims 49 to 51, wherein the glucose metabolism-related gene is insulin gene or glucose transporter 2 gene.

53. A method for regulating production of a gene product of a glucose metabolism-related gene, wherein the method comprises changing the degree of degradation of a glucose metabolism-related gene by calcium concentration.

54. A method for regulating production of a gene product of a glucose metabolism-related gene, wherein the method comprises changing the degree of degradation of at least one member selected from the group consisting of hepatocyte nuclear factor 4α, hepatocyte nuclear factor 1α and insulin promoter factor I by calcium concentration.

55. A method for enhancing production of a gene product of a glucose metabolism-related gene, wherein the method comprises using the method for inhibiting the degradation of a transcription factor according to any one of claims 8 to 24.

56. A method for enhancing production of a gene product of a gene on which at least one member selected from the group consisting of hepatocyte nuclear factor 4α, hepatocyte nuclear factor 1α and insulin promoter factor 1 acts as a transcription factor, wherein the method comprises using the method for inhibiting the degradation of a transcription factor according to any one of claims 8 to 24.

57. A method for enhancing production of a gene product of the insulin gene and/or glucose transporter 2 gene, wherein the method comprises using the method for inhibiting the degradation of a transcription factor according to any one of claims 8 to 24.

58. A method for preventing and/or treating a disease attributable to the degradation of a transcription factor of a glucose metabolism-related gene, wherein the method comprises using the method for inhibiting the degradation of a transcription factor according to any one of claims 8 to 24.

59. A method for preventing and/or treating a disease attributable to the degradation of at least one member selected from the group consisting of hepatocyte nuclear factor 4α, hepatocyte nuclear factor 1α and insulin promoter factor 1, wherein the method comprises using the method for inhibiting the degradation of a transcription factor according to any one of claims 8 to 24.

60. A method for preventing and/or treating a disease attributable to a decrease in a gene product of a glucose metabolism-related gene, wherein the method comprises using the method for inhibiting the degradation of a transcription factor according to any one of claims 8 to 24.

61. A method for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the method comprises using the method for inhibiting the degradation of a transcription factor according to any one of claims 8 to 24.

62. A method for preventing and/or treating a disease attributable to a decrease in a gene product of the insulin gene and/or glucose transporter 2 gene, wherein the method comprises using the method for inhibiting the degradation of a transcription factor according to any one of claims 8 to 24.

63. A method for preventing and/or treating diabetes, wherein the method comprises using the method for inhibiting the degradation of a transcription factor according to any one of claims 8 to 24.

64. A method for enhancing production of a gene product of a glucose metabolism-related gene, wherein the method comprises using the agent for inhibiting the degradation of a transcription factor according to any one of claims 31 to 44.

65. A method for enhancing production of a gene product of a gene on which at least one member selected from the group consisting of hepatocyte nuclear factor 4α, hepatocyte nuclear factor 1α and insulin promoter factor 1 acts as a transcription factor, wherein the method comprises using the agent for inhibiting the degradation of a transcription factor according to any one of claims 31 to 44.

66. A method for enhancing production of a gene product of the insulin gene and/or glucose transporter 2 gene, wherein the method comprises using the agent for inhibiting the degradation of a transcription factor according to any one of claims 31 to 44.

67. A method for preventing and/or treating a disease attributable to the degradation of a transcription factor of a glucose metabolism-related gene, wherein the method comprises using the agent for inhibiting the degradation of a transcription factor according to any one of claims 31 to 44.

68. A method for preventing and/or treating a disease attributable to the degradation of at least one member selected from the group consisting of hepatocyte nuclear factor 4α, hepatocyte nuclear factor 1α and insulin promoter factor 1, wherein the method comprises using the agent for inhibiting the degradation of a transcription factor according to any one of claims 31 to 44.

69. A method for preventing and/or treating a disease attributable to a decrease in a gene product of a glucose metabolism-related gene, wherein the method comprises using the agent for inhibiting the degradation of a transcription factor according to any one of claims 31 to 44.

70. A method for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the method comprises using the agent for inhibiting the degradation of a transcription factor according to any one of claims 31 to 44.

71. A method for preventing and/or treating a disease attributable to a decrease in a gene product of the insulin gene and/or glucose transporter 2 gene, wherein the method comprises using the agent for inhibiting the degradation of a transcription factor according to any one of claims 31 to 44.

72. A method for preventing and/or treating diabetes, wherein the method comprises using the agent for inhibiting the degradation of a transcription factor according to any one of claims 31 to 44.

73. An agent for enhancing production of a gene product of a glucose metabolism-related gene, wherein the agent contains an effective dose of the agent for inhibiting the degradation of a transcription factor according to any one of claims 31 to 44.

74. An agent for enhancing production of a gene product of a gene on which at least one member selected from the group consisting of hepatocyte nuclear factor 4α, hepatocyte nuclear factor 1α and insulin promoter factor 1 acts as a transcription factor, wherein the agent contains an effective dose of the agent for inhibiting the degradation of a transcription factor according to any one of claims 31 to 44.

75. An agent for enhancing production of a gene product of the insulin gene and/or glucose transporter 2 gene, wherein the agent contains an effective dose of the agent for inhibiting the degradation of a transcription factor according to any one of claims 31 to 44.

76. A pharmaceutical composition which contains an effective dose of the agent for inhibiting the degradation of a transcription factor according to any one of claims 31 to 44.

77. An agent for preventing and/or treating a disease attributable to the degradation of a transcription factor of a glucose metabolism-related gene, wherein the agent contains an effective dose of the agent for inhibiting the degradation of a transcription factor according to any one of claims 31 to 44.

78. An agent for preventing and/or treating a disease attributable to the degradation of at least one member selected from the group consisting of hepatocyte nuclear factor 4α, hepatocyte nuclear factor 1α and insulin promoter factor 1, wherein the agent contains an effective dose of the agent for inhibiting the degradation of a transcription factor according to any one of claims 31 to 44.

79. An agent for preventing and/or treating a disease attributable to a decrease in a gene product of a glucose metabolism-related gene, wherein the agent contains an effective dose of the agent for inhibiting the degradation of a transcription factor according to any one of claims 31 to 44.

80. An agent for preventing and/or treating a disease attributable to a decrease in a gene product of a gene on which at least one member selected from the group consisting of hepatocyte nuclear factor 4α, hepatocyte nuclear factor 1α and insulin promoter factor 1 acts as a transcription factor, wherein the agent contains an effective dose of the agent for inhibiting the degradation of a transcription factor according to any one of claims 31 to 44.

81. An agent for preventing and/or treating a disease attributable to a decrease in a gene product of insulin gene and/or glucose transporter 2 gene, wherein the agent contains an effective dose of the agent for inhibiting the degradation of a transcription factor according to any one of claims 31 to 44.

82. An agent for preventing and/or treating diabetes, wherein the agent contains an effective dose of the agent for inhibiting the degradation of a transcription factor according to any one of claims 31 to 44.

83. A method for preventing and/or treating liver adenoma or hepatocellular carcinoma, wherein the method comprises using the degradation inhibition method according to claim 23.

84. A method for preventing and/or treating liver adenoma or hepatocellular carcinoma, wherein the method comprises using the degradation inhibitory agent according to claim 43.

85. An agent for preventing and/or treating liver adenoma or hepatocellular carcinoma, wherein the agent contains an effective dose of the degradation inhibitory agent according to claim 43.

86. A method for identifying a compound that inhibits the degradation of a transcription factor of a glucose metabolism-related gene by calpain, wherein the method comprises contacting calpain and/or the transcription factor with a test compound under conditions that allow the cleavage of the transcription factor by calpain; and determining whether the test compound inhibits the cleavage of the transcription factor by calpain, by introducing a system using a signal and/or a marker capable of detecting the degradation of the transcription factor by calpain and detecting the presence, absence or change of the signal and/or the marker.

87. A method for identifying a compound that inhibits the degradation of a transcription factor of a glucose metabolism-related gene by calpain, wherein the method comprises contacting calpain and/or the transcription factor with a test compound under conditions that allow the cleavage of the transcription factor by calpain; and determining whether the test compound inhibits the cleavage of the transcription factor by calpain, by introducing a system using a signal and/or a marker capable of detecting the amount of the transcription factor or the amount of a degradation product of the transcription factor and detecting the presence, absence or change of the signal and/or the marker.

88. A method for identifying a compound that inhibits the degradation of a transcription factor of a glucose metabolism-related gene by calpain, wherein the method comprises contacting calpain and/or the transcription factor with a test compound under conditions that allow the binding of calpain to the transcription factor; and determining whether the test compound inhibits the binding of calpain to the transcription factor, by introducing a system using a signal and/or a marker capable of detecting the binding of calpain to the transcription factor and detecting the presence, absence or change of the signal and/or the marker.

89. The identification method according to any one of claims 86 to 88, wherein calpain is m-calpain or µ-calpain.

90. The identification method according to any one of claims 86 to 89, wherein the transcription factor of a glucose metabolism-related gene is at least one member selected from the group consisting of hepatocyte nuclear factor 4α, hepatocyte nuclear factor 1α and insulin promoter factor 1.

91. A compound identified by the identification method according to any one of claims 86 to 90.

92. A reagent kit containing at least one member selected from the group consisting of calpain, a polynucleotide encoding calpain, and a vector containing a polynucleotide encoding calpain; and at least one member selected from the group consisting of a transcription factor of a glucose metabolism-related gene that is degraded by calpain, a polynucleotide encoding the transcription factor, and a vector containing the polynucleotide.

93. A reagent kit containing at least one member selected from the group consisting of calpain, a polynucleotide encoding calpain, and a vector containing a polynucleotide encoding calpain; and at least one member selected from the group consisting of hepatocyte nuclear factor 4α, hepatocyte nuclear factor 1α, insulin promoter factor 1, a polynucleotide encoding any of these, and a vector containing the polynucleotide.

94. The reagent kit according to claim 92 or 93, wherein calpain is m-calpain or µ-calpain.
